# EUROPÄISCHE PATENTSCHRIFT

(11) **EP 2 334 629 B1**
(45) Veröffentlichungstag und Bekanntmachung des Hinweises auf die Patenterteilung: **10.08.2016**
(21) Anmeldenummer: 09782183.9
(22) Anmeldetag: 26.08.2009
(51) Int. Cl.: C07C 49/11

(54) **VERFAHREN ZUR HERSTELLUNG VON ZYKLISCHEN KETONEN**
PROCESS FOR PREPARING CYCLIC KETONES
PROCÉDÉ DE PRÉPARATION DE CÉTONES CYCLIQUES

(30) Priorität: 29.08.2008 EP 08163319
(43) Veröffentlichungstag der Anmeldung: 22.06.2011
(73) Patentinhaber: BASF SE, 67056 Ludwigshafen am Rhein (DE)
(72) Erfinder: TELES, Joaquim, Henrique, 67166 Otterstadt (DE); RUPPEL, Wilhelm, 68163 Mannheim (DE); WEGERLE, Ulrike, 67550 Worms (DE); MEIER, Anton, 67134 Birkenheide (DE); GENGER, Thomas, 67245 Lambsheim (DE); SCHELPER, Michael, 67065 Ludwigshafen (DE); RESCH, Peter, 67310 Hettenleidelheim (DE)
(86) Internationale Anmeldenummer: PCT/EP2009/060956
(87) Internationale Veröffentlichungsnummer: WO 2010/023211

(56) Entgegenhaltungen:
- WO-A1-98/15514
- WO-A1-2008/148661
- US-A1- 2005 203 316
- BRIDSON-JONES F S ET AL: "OXIDATION OF ORGANIC COMPOUNDS BY NITROUS OXIDE" JOURNAL OF THE CHEMICAL SOCIETY. ABSTRACTS,, 1. Januar 1951 (1951-01-01), Seiten 2999-3008, XP009045346 ISSN: 0590-9791 in der Anmeldung erwähnt

## Beschreibung

Die vorliegende Erfindung betrifft ein kontinuierliches Verfahren zur Herstellung von wenigstens einem monozyklischen Keton mit 4 bis 20 Kohlenstoffatomen durch Umsetzung bei einer Temperatur von 170 bis 340 °C eines Gemischs G1 enthaltend wenigstens ein monozyklisches Olefin mit 4 bis 20 Kohlenstoffatomen mit einem Gemisch G2 enthaltend wenigstens Distickstoffmonoxid, dadurch gekennzeichnet, dass diese Umsetzung adiabatisch durchgeführt wird und das Gemisch G1 und/oder G2 vor der Umsetzung zu wenigstens einem monozyklischen Keton mit 4 bis 20 Kohlenstoffatomen auf eine Temperatur von 170 bis 270 °C vorgewärmt werden, wobei die zum Vorwärmen von Gemisch G1 und/oder G2, notwendige Wärmeenergie zumindest teilweise aus dem Produktstrom des Verfahrens entnommen wird.

Verfahren zur Herstellung von Cyclopentanon sind grundsätzlich aus dem Stand der Technik bekannt. Ebenfalls bekannt ist, dass Cyclopentanon durch Umsetzung von Cyclopenten mit Distickstoffmonoxid erhalten werden kann. Die Herstellung von Cyclopentanon durch Oxidation von Cyclopenten mit Distickstoffmonoxid ist eine sehr selektive Reaktion, die stark exotherm ist.

So offenbart die GB 649,680 die Umsetzung von Alkenen wie beispielsweise Cyclohexen mit Distickstoffmonoxid, um die entsprechenden Cycloketone, beispielsweise Cyclohexanon, zu erhalten. Die Reaktion wird bei einer Temperatur von 200 bis 300 °C und einem Druck von 100 bis 500 bar in der Flüssigphase durchgeführt. In der genannten Schrift wird nicht offenbart, dass zyklische Olefine mit Distickstoffmonoxid unter adiabatischen Bedingungen umgesetzt werden.

F. S. Bridson-Jones et al. beschreiben in J. Chem. Soc., S. 2999-3008 (1951) die Umsetzung von Olefinen mit Distickstoffmonoxid, wobei beispielsweise Cyclohexen zu Cyclohexanon umgesetzt wird. Das Verfahren gemäß diesem Dokument wird bei einer Temperatur von beispielsweise 300 °C und einem Druck von 500 bar in einem Autoklaven durchgeführt. F. S. Bridson-Jones et al. offenbaren nicht, dass zyklische Olefine mit Distickstoffmonoxid unter adiabatischen Bedingungen umgesetzt werden.

Die Synthese von Carbonylverbindungen aus Alkenen mit Distickstoffmonoxid wird auch in verschiedenen internationalen Patentanmeldungen beschrieben. So offenbart die WO 03/078370 ein Verfahren zur Herstellung von Carbonylverbindungen aus aliphatischen Alkenen mit Distickstoffmonoxid. Die Umsetzung wird bei Temperaturen im Bereich von 20 bis 350 °C und Drücken von 0,01 bis 100 bar durchgeführt. WO 03/078374 offenbart ein entsprechendes Verfahren zur Herstellung von Cyclohexanon. Gemäß der WO 03/078372 werden zyklische Ketone mit 4 bis 5 C-Atomen hergestellt. Gemäß der WO 03/078375 werden unter diesen Verfahrensbedingungen zyklische Ketone aus zyklischen Alkenen mit 7 bis 20 C-Atomen hergestellt. WO 03/078371 offenbart ein Verfahren zur Herstellung substituierter Ketone aus substituierten Alkenen.

WO 04/000777 offenbart ein Verfahren zur Umsetzung von Di- und Polyalkenen mit Distickstoffmonoxid zu den entsprechenden Carbonylverbindungen. Ein adiabatisches Verfahren zur Herstellung zyklischer Ketone aus den entsprechenden zyklischen Olefinen wird in den genannten internationalen Anmeldungen nicht offenbart.

US 4,806,692 offenbart ein Verfahren zur Herstellung von Sauerstoff enthaltenden organischen Verbindungen aus Olefinen. Insbesondere wird die Oxidation von zyklischen Olefinen unter milden Bedingungen offenbart, um entsprechende zyklische Ketone zu erhalten. Eine solche Oxidation erfolgt gemäß US 4,806,692 in Gegenwart von Palladium-Katalysatoren bei einer Temperatur von 80 °C oder weniger und Atmosphärendruck. US 4,806,692 offenbart kein Verfahren zur Herstellung von zyklischen Ketonen aus den entsprechenden zyklischen Olefinen durch Umsetzung mit Distickstoffmonoxid unter adiabatischen Bedingungen.

US 7,282,612 B2 offenbart ein Verfahren zur Herstellung von monozyklischen Ketonen mit 4 oder 5 Kohlenstoffatomen durch Umsetzung der entsprechenden zyklischen Alkene mit 4 bzw. 5 Kohlenstoffatomen mit Distickstoffmonoxid, gegebenenfalls in Mischung mit einem inerten Gas bei einer Temperatur von 20 bis 300 °C und einem Distickstoffmonoxid-Druck von 0,01 bis 10 bar. Ein adiabatisch durchgeführtes Verfahren wird in US 7,282,612 B2 nicht offenbart.

RU 2002106986 offenbart ein Verfahren zur Herstellung von monozyklischen Ketonen mit 4 oder 5 Kohlenstoffatomen durch Oxidation von Cyclobuten oder Cyclopenten mit Distickstoffmonoxid, ebenfalls bei einem Distickstoffmonoxid-Druck von 0,01 bis 100 bar und einer Temperatur von 20 bis 300 C°.

Die Umsetzung von monozyklischen Olefinen mit Distickstoffmonoxid, um die entsprechenden Ketone zu erhalten, ist stark exotherm. Des Weiteren sind Mischungen von Distickstoffmonoxid mit organischen Verbindungen mit einer hohen Konzentration an Distickstoffmonoxid explosionsgefährdet. Daher ist es für ein entsprechendes Verfahren gemäß dem Stand der Technik notwendig, aufwendige und kostenintensive Vorrichtungen zur Wärmeabfuhr der exothermen Reaktion bereitzustellen. Des Weiteren müssen die Reaktoren für die hohen Drücke und Temperaturen ausgelegt sein.

Aufgabe der vorliegenden Erfindung ist es somit, ein Verfahren zur Herstellung von monozyklischen Ketonen mit 4 bis 20 Kohlenstoffatomen bereitzustellen, welches sich dadurch auszeichnet, dass keine aufwendigen und damit kostenintensiven Vorrichtungen bereitgestellt werden müssen.

Eine weitere Aufgabe der vorliegenden Erfindung ist es, ein entsprechendes Verfahren bereitzustellen, mit dem monozyklische Ketone, insbesondere Cyclopentanon und/oder Cyclohexanon, in hoher Ausbeute und möglichst hoher Reinheit zugänglich sind.

Diese Aufgaben werden erfindungsgemäß gelöst durch ein kontinuierliches Verfahren zur Herstellung von wenigstens einem monozyklischen Keton mit 4 bis 20 Kohlenstoffatomen durch Umsetzung bei einer Temperatur von 170 bis 340 °C eines Gemischs G1 enthaltend wenigstens ein monozyklisches Olefin mit 4 bis 20 Kohlenstoffatomen mit einem Gemisch G2 enthaltend wenigstens Distickstoffmonoxid, dadurch gekennzeichnet, dass diese Umsetzung adiabatisch durchgeführt wird und das Gemisch G1 und/oder G2 vor der Umsetzung zu wenigstens einem monozyklischen Keton mit 4 bis 20 Kohlenstoffatomen auf eine Temperatur von 170 bis 270 °C vorgewärmt werden, wobei die zum Vorwärmen von Gemisch G1 und/oder G2, notwendige Wärmeenergie zumindest teilweise aus dem Produktstrom des Verfahrens entnommen wird.

Im Rahmen der vorliegenden Erfindung wird unter einer adiabatisch geführten Umsetzung eine Umsetzung verstanden, bei der während der Reaktion im Wesentlichen kein Wärmeaustausch zwischen Reaktorinhalt und Umgebung stattfindet. Bevorzugt wird im Rahmen der vorliegenden Erfindung unter einer adiabatisch geführten Umsetzung eine Umsetzung verstanden, bei der bevorzugt weniger als 10 %, besonders bevorzugt weniger als 5% der erzeugten Wärme an die Umgebung abgegeben werden.

Bislang bekannte Verfahren zur Herstellung von zyklischen Ketonen aus den entsprechenden Olefinen und Distickstoffmonoxid weisen den Nachteil auf, dass durch die stark exotherme Reaktion von Olefin und Distickstoffmonoxid eine große Menge an Wärme erzeugt wird, die von dem Reaktionsgemisch bzw. dem Reaktor, in dem die Reaktion stattfindet, abgeführt werden muss. Dies führt zu hohen Material- und somit Investitionskosten für den Reaktor. Eine baulich einfache Ausführung des Reaktors zur Herstellung von monozyklischen Ketonen aus den entsprechenden Olefinen und Distickstoffmonoxid war bislang nicht möglich.

Es wurde gefunden, dass das beschriebene Problem dadurch gelöst werden kann, dass die stark exotherme Reaktion adiabatisch durchgeführt wird, d. h. dass die während der Reaktion erzeugte Wärme im Wesentlichen im System verbleibt und nicht nach außen abgeführt wird. Dadurch, dass die während der Reaktion erzeugte Reaktionswärme im System verbleibt, vereinfacht sich der Reaktoraufbau und die Verfahrensführung sehr stark, da kühlende bzw. wärmedispergierende Elemente nicht baulich im Reaktor realisiert werden müssen.

In einer bevorzugten Ausführungsform wird das erfindungsgemäße Verfahren durchgeführt, indem die Gemische G1 und G2 in einem thermisch gegenüber der Umgebung isolierten Reaktor umgesetzt werden, wobei die bei der exothermen Reaktion erzeugte Wärmeenergie im Wesentlichen im Reaktor verbleibt und nicht nach außen abgeführt wird.

Erfindungsgemäß kann die erzeugte Reaktionswärme bevorzugt durch die Umsätze der einzelnen Edukte eingestellt werden. Die Umsätze der einzelnen Edukte können wiederum durch die Verweilzeit, durch die Eingangstemperatur des Eduktgemisches (Tₑᵢₙ), durch den Reaktionsdruck und durch die Konzentrationen der einzelnen Edukte im Eduktgemisch beeinflusst werden. Somit ist es erfindungsgemäß möglich, beispielsweise durch Wahl der genannten Parameter in Verbindung mit einem geeigneten Reaktor das Verfahren adiabatisch, d. h. im Wesentlichen ohne Zu- und/oder Abfuhr von Wärmeenergie in bzw. aus der Reaktionsmischung innerhalb des Reaktors, durchzuführen.

Bei einer adiabatischen Verfahrensführung ist die Differenz zwischen der Temperatur der Produkte (Tₐᵤₛ) und der Temperatur der Edukte (Tₑᵢₙ) als adiabatische Temperaturerhöhung (T_{adiab}) definiert. In einer bevorzugten Ausführungsform des erfindungsgemäßen Verfahrens beträgt T_{adiab} zwischen 10 und 140 °C, besonders bevorzugt zwischen 20 und 125 °C und ganz besonders bevorzugt zwischen 25 und 100 °C.

Demnach betrifft die vorliegende Erfindung in einer bevorzugten Ausführungsform auch ein wie oben beschriebenes Verfahren zur Herstellung von wenigstens einem monozyklischen Keton mit 4 bis 20 Kohlenstoffatomen durch Umsetzung eines Gemischs G1 enthaltend wenigstens ein monozyklisches Olefin mit 4 bis 20 Kohlenstoffatomen mit einem Gemisch G2 enthaltend wenigstens Distickstoffmonoxid, wobei die adiabatische Temperaturerhöhung im Reaktor zwischen 10 und 140 °C, besonders bevorzugt zwischen 20 und 125 °C und ganz besonders bevorzugt zwischen 25 und 100 °C, beträgt.

Das erfindungsgemäße Verfahren kann in einer bevorzugten Ausführungsform derart durchgeführt werden, dass die oben genannten Parameter so eingestellt werden, dass die durch die Reaktion erzeugte Reaktionswärme die Wärme ist, welche notwendig ist, damit das Produktionsgemisch den Reaktor mit einer Temperatur (Tₐᵤₛ) verlässt, die noch deutlich unter der Onset-Temperatur für deren Zersetzung liegt. Im Rahmen der vorliegenden Erfindung wird die Onset-Temperatur definiert als die Temperatur, ab der in einem Differential-Scanning-Calorimetry-Versuch (DSC-Versuch) des Produktgemisches mit einer Temperaturerhöhungsrate von mindestens 0,1 K/min eine deutliche exotherme Reaktion zu verzeichnen ist.

Demnach betrifft die vorliegende Erfindung in einer bevorzugten Ausführungsform auch ein wie oben beschriebenes Verfahren zur Herstellung von wenigstens einem monozyklischen Keton mit 4 bis 20 Kohlenstoffatomen durch Umsetzung eines Gemischs G1 enthaltend wenigstens ein monozyklisches Olefin mit 4 bis 20 Kohlenstoffatomen mit einem Gemisch G2 enthaltend wenigstens Distickstoffmonoxid, wobei die Reaktorausgangstemperatur unterhalb der Onset-Temperatur für die Zersetzung des Produktgemisches liegt.

Das erfindungsgemäße Verfahren kann in einer besonders bevorzugten Ausführungsform derart durchgeführt werden, dass die oben genannten Parameter so eingestellt werden, dass die durch die Reaktion erzeugte Reaktionswärme die Wärme ist, welche notwendig ist, damit das Produktionsgemisch den Reaktor mit einer Temperatur (Tₐᵤₛ) verlässt, die mindestens 10 K unterhalb der Temperatur liegt, bei der die adiabatische Induktionszeit genau 24 Stunden beträgt. Die adiabatische Induktionszeit in Abhängigkeit der Temperatur kann auf an sich bekannte Weise aus den Daten von DSC-Experimenten mit unterschiedlichen Aufheizraten abgeleitet werden.

Demnach betrifft die vorliegende Erfindung in einer bevorzugten Ausführungsform auch ein wie oben beschriebenes Verfahren zur Herstellung von wenigstens einem monozyklischen Keton mit 4 bis 20 Kohlenstoffatomen durch Umsetzung eines Gemischs G1 enthaltend wenigstens ein monozyklisches Olefin mit 4 bis 20 Kohlenstoffatomen mit einem Gemisch G2 enthaltend wenigstens Distickstoffmonoxid, wobei die Reaktorausgangstemperatur mindestens 10 K unterhalb der Temperatur liegt, bei der die adiabatische Induktionszeit des Produktgemisches 24 Stunden beträgt.

Dabei ist es erfindungsgemäß möglich, dass beide Edukte, d. h. das wenigstens eine monozyklische Olefin mit 4 bis 20 Kohlenstoffatomen und Distickstoffmonoxid, die gleiche oder verschiedene Eingangstemperatur(en) aufweisen. Relevant ist im Rahmen der vorliegenden Erfindung die Reaktoreingangstemperatur des Eduktgemischs, also die Temperatur, die sich einstellt, wenn alle Eduktströme zusammengemischt werden.

In einer bevorzugten Ausführungsform der Erfindung beträgt die Reaktoreingangstemperatur des Eduktgemischs (Tₑᵢₙ) 170 bis 270 °C, besonders bevorzugt 200 bis 260 °C, beispielsweise 220 bis 250 °C.

Demnach betrifft die vorliegende Erfindung in einer bevorzugten Ausführungsform auch ein Verfahren zur Herstellung von wenigstens einem monozyklischen Keton mit 4 bis 20 Kohlenstoffatomen durch Umsetzung eines Gemischs G1 enthaltend wenigstens ein monozyklisches Olefin mit 4 bis 20 Kohlenstoffatomen mit einem Gemisch G2 enthaltend wenigstens Distickstoffmonoxid, wobei diese Umsetzung adiabatisch durchgeführt wird und die Reaktoreingangstemperatur des Eduktgemischs (Tₑᵢₙ) 170 bis 270 °C beträgt.

Die Temperatur, die die Edukte am Reaktoreingang aufweisen, entspricht bevorzugt auch der minimalen Temperatur, bei der im erfindungsgemäßen Verfahren der gewünschte Umsatz noch in einer technisch realisierbaren Reaktorgröße erzielt werden kann. Somit beträgt die minimale Temperatur, bei der im erfindungsgemäßen Verfahren der gewünschte Umsatz noch in einer technisch realisierbaren Reaktorgröße erzielt werden kann, im Allgemeinen wenigstens 170 °C, bevorzugt wenigstens 200 °C.

Die maximale Reaktorausgangstemperatur (Tₐᵤₛ) des Produktgemischs, bei der das erfindungsgemäße Verfahren durchgeführt werden kann, beträgt im Allgemeinen höchstens 340 °C, bevorzugt höchstens 320 °C, besonders bevorzugt höchstens 300 °C. Die maximale Reaktorausgangstemperatur (Tₐᵤₛ) wird erfindungsgemäß so gewählt, dass bevorzugt keine thermische Zersetzung des gebildeten Produktes bzw. der nicht umgesetzten Edukte stattfindet.

Somit wird das erfindungsgemäße Verfahren bei einer Temperatur von 170 bis 340 °C, bevorzugt 200 bis 320 °C, durchgeführt, wobei die erstgenannte Temperatur die Reaktoreingangstemperatur (Tₑᵢₙ) des Eduktgemischs und die zweitgenannte Temperatur die Reaktorausgangstemperatur (Tₐᵤₛ) des Produktgemischs ist.

In einer bevorzugten Ausführungsform wird das erfindungsgemäße Verfahren bei einem Reaktionsdruck von 60 bis 500 bar, besonders bevorzugt von 80 bis 325 bar, besonders bevorzugt von 90 bis 180 bar, beispielsweise bei 100 bis 150 bar, durchgeführt.

Demnach betrifft die vorliegende Erfindung in einer bevorzugten Ausführungsform auch ein Verfahren zur Herstellung von wenigstens einem monozyklischen Keton mit 4 bis 20 Kohlenstoffatomen durch Umsetzung eines Gemischs G1 enthaltend wenigstens ein monozyklisches Olefin mit 4 bis 20 Kohlenstoffatomen mit einem Gemisch G2 enthaltend wenigstens Distickstoffmonoxid, wobei diese Umsetzung adiabatisch durchgeführt wird und der Reaktionsdruck 60 bis 500 bar beträgt.

Das erfindungsgemäße Verfahren kann in einer weiteren Ausführungsform derart durchgeführt werden, dass das molare Verhältnis zwischen den Substraten, d. h. dem wenigstens einen monozyklischen Olefin mit 4 bis 20 Kohlenstoffatomen und Distickstoffmonoxid einen geeigneten Wert hat, so dass die durch die Reaktion erzeugte Reaktionswärme genau die Wärme ist, welche bei einer entsprechenden Reaktoreingangstemperatur (Tₑᵢₙ) des Eduktgemischs und bei Vollumsatz des im Unterschuss vorliegenden Eduktes, bevorzugt Distickstoffmonoxid, eine Reaktorausgangstemperatur (Tₐᵤₛ) des Produktgemischs ergibt, die unterhalb der oben genannten maximalen Temperaturen von 340 °C, bevorzugt 320 °C, besonders bevorzugt 300 °C, liegt.

In einer bevorzugten Ausführungsform beträgt das molare Verhältnis zwischen Distickstoffmonoxid und dem wenigstens einen monozyklischen Olefin zwischen 0,02 und 0,3, besonders bevorzugt zwischen 0,05 und 0,25, und ganz besonders bevorzugt zwischen 0,08 und 0,2. Erfindungsgemäß wird unter dem "molaren Verhältnis der Edukte" der Quotient der Stoffmengen der Edukte verstanden. Da die Stoffmengen der Edukte jeweils die Einheit mol tragen, ist der Quotient dieser Stoffmengen einheitenlos.

Demnach betrifft die vorliegende Erfindung in einer bevorzugten Ausführungsform auch ein Verfahren zur Herstellung von wenigstens einem monozyklischen Keton mit 4 bis 20 Kohlenstoffatomen durch Umsetzung eines Gemischs G1 enthaltend wenigstens ein monozyklisches Olefin mit 4 bis 20 Kohlenstoffatomen mit einem Gemisch G2 enthaltend wenigstens Distickstoffmonoxid, wobei diese Umsetzung adiabatisch durchgeführt wird und das molare Verhältnis zwischen Distickstoffmonoxid und dem wenigstens einen monozyklischen Olefin zwischen 0,02 und 0,3, bevorzugt zwischen 0,05 und 0,25 liegt.

In einer weiteren bevorzugten Ausführungsform liegt in dem erfindunsgemäßen Verfahrens der Umsatz bezüglich Distickstoffmonoxid bei 80 bis 100%, besonders bevorzugt bei 90 bis 99%, ganz besonders bevorzugt bei 90 bis 96%.

Demnach betrifft die vorliegende Erfindung in einer bevorzugten Ausführungsform auch ein Verfahren zur Herstellung von wenigstens einem monozyklischen Keton mit 4 bis 20 Kohlenstoffatomen wie oben beschrieben, dadurch gekennzeichnet, dass der Umsatz bezüglich Distickstoffmonoxid bei 80 bis 100% liegt.

In einer ganz besonders bevorzugten Ausführungsform betrifft die vorliegende Erfindung auch ein Verfahren zur Herstellung von wenigstens einem monozyklischen Keton mit 4 bis 20 Kohlenstoffatomen durch Umsetzung eines Gemischs G1 enthaltend wenigstens ein monozyklisches Olefin mit 4 bis 20 Kohlenstoffatomen mit einem Gemisch G2 enthaltend wenigstens Distickstoffmonoxid, wobei diese Umsetzung adiabatisch durchgeführt wird, das Verfahren bei einer Temperatur von 170 bis 340 °C durchgeführt wird, der Reaktionsdruck 60 bis 500 bar beträgt, das molare Verhältnis zwischen Distickstoffmonoxid und dem wenigstens einen monozyklischen Olefin zwischen 0,05 und 0,25 beträgt und der Umsatz bezüglich Distickstoffmonoxid bei 80 bis 100% liegt.

Das erfindungsgemäße Verfahren kann in allen dem Fachmann bekannten Reaktoren durchgeführt werden, die für eine adiabatische Reaktionsführung geeignet sind, beispielsweise in einem Rohrreaktor. Um eine adiabatische Reaktionsführung zu gewährleisten, ist es beispielsweise notwendig, dass der Reaktor ausreichend gegenüber der Umgebung isoliert ist, so dass im Wesentlichen keine Reaktionswärme an die Umgebung abgegeben wird und somit der eigentlichen Umsetzung nicht mehr zur Verfügung steht. In einer besonders bevorzugten Ausführungsform wird die durch die Reaktion erzeugte Wärme durch den Produktstrom aus dem Reaktor ausgetragen.

Dabei ist es erfindungsgemäß auch möglich, mehrere Reaktoren zu verwenden, die parallel oder in Serie geschaltet sein können.

Das erfindungsgemäße Verfahren wird kontinuierlich durchgeführt.

Der Reaktorraum des erfindungsgemäß einsetzbaren Reaktors kann leer sein oder gegebenenfalls durch geeignete Einbauten segmentiert sein. Im Allgemeinen weist der Reaktor ein für eine adiabatisch geführte Reaktion geeignetes Strömungsprofil auf. In dem für das erfindungsgemäße Verfahren einzusetzenden Reaktor findet bevorzugt im Wesentlichen keine Rückvermischung statt. Bevorzugt hat der Reaktor eine Verweilzeitverteilung, die der einer Rührkesselkaskade mit mindestens 8 Rührkesseln entspricht. Besonders bevorzugt hat der Reaktor eine Verweilzeitverteilung, die der einer Rührkesselkaskade mit mindestens 12 Rührkesseln entspricht. Das für das erfindungsgemäße Verfahren bevorzugte Strömungsprofil der Reaktionsmischung ist abhängig vom eingesetzten Reaktor und kann gegebenenfalls durch geeignete, dem Fachmann bekannte Einbauten, beispielsweise Lochbleche, oder durch Füllung des Reaktors mit einer geeigneten Schüttung, entsprechend eingestellt werden.

Bevorzugt wird für das erfindungsgemäße Verfahren ein Rohrreaktor mit einem Verhältnis von Länge zu Durchmesser von größer 1 eingesetzt. Besonders bevorzugt enthält der Reaktor mindestens Lochbleche zur Verringerung der Rückvermischung.

Es ist erfindungsgemäß möglich, dass der Reaktor liegend oder stehend betrieben wird, bevorzugt stehend. Ein stehender Reaktor kann von unten nach oben oder von oben nach unten von dem Reaktionsgemisch durchströmt werden. Bevorzugt wird das erfindungsgemäße Verfahren in einem stehenden Reaktor, der von unten nach oben von dem Reaktionsgemisch durchströmt wird, durchgeführt.

Ein für die kontinuierliche Verfahrensführung besonders geeigneter Reaktor ist beispielsweise ein Rohrreaktor, der bevorzugt ausreichend isoliert ist. Entsprechende Rohrreaktoren sind dem Fachmann bekannt.

Es ist erfindungsgemäß möglich, dass die Eduktströme, bevorzugt Gemisch G1 und Gemisch G2, dem Reaktor separat zugeführt werden. Es ist erfindungsgemäß auch möglich und bevorzugt, dass die Eduktströme bereits vorgemischt dem Reaktor zugeführt werden.

In einer besonders bevorzugten Ausführungsform werden in dem erfindungsgemäßen Verfahren die Eduktströme, bevorzugt Gemisch G1 und Gemisch G2, beispielsweise mit Hilfe einer geeigneten Mischvorrichtung, beispielsweise einem statischen Mischer, vor dem Reaktoreingang gemischt.

Die Temperatur der Gemische G1 und G2 wird so gewählt, dass die Temperatur des gemischten Eduktstromes die gewünschte Temperatur Tₑᵢₙ aufweist. Besonders bevorzugt wird lediglich Gemisch G1 vorgeheizt und vor dem Reaktor in einer geeigneten Mischvorrichtung mit einem nicht vorgeheizten Gemisch G2 gemischt, wobei die Temperatur, auf die das Gemisch G1 aufgeheizt wird, so gewählt wird, dass die Temperatur des gemischten Eduktstromes der gewünschten Temperatur Tₑᵢₙ entspricht.

Die Eduktströme, bevorzugt Gemisch G1 und/oder G2, besonders bevorzugt nur Gemisch G1, können nach allen dem Fachmann bekannten Verfahren vor der Umsetzung zu wenigstens einem monozyklischen Keton mit 4 bis 20 Kohlenstoffatomen auf eine Temperatur von bevorzugt 170 bis 270 °C, besonders bevorzugt 200 bis 260 °C, beispielsweise 220 bis 250 °C, vorgewärmt werden, beispielsweise durch eine externe Wärmequelle, beispielsweise Wasserdampf, in einem dem Fachmann bekannten Wärmetauscher, der erfindungsgemäß als Vorwärmer fungiert. Das Vorwärmen der Eduktströme erfolgt erfindungsgemäß außerhalb des Reaktors in einem geeigneten Wärmeaustauscher.

Die vorliegende Erfindung betrifft somit auch ein Verfahren, wie oben beschrieben, dadurch gekennzeichnet, dass das Gemisch G1 und/oder G2 vor der Umsetzung zu wenigstens einem monozyklischen Keton mit 4 bis 20 Kohlenstoffatomen auf eine Temperatur von 170 bis 270 °C vorgewärmt werden.

In einer besonders bevorzugten Ausführungsform des erfindungsgemäßen Verfahrens wird zumindest ein Teil des Gemisches G1 auf eine entsprechende Temperatur vorgewärmt, bevor es mit Gemisch G2, bevorzugt kurz vor dem Reaktor oder im Reaktor, kontaktiert wird. Somit kann beispielsweise vermieden werden, dass die erfindungsgemäße Reaktion schon außerhalb des eigentlichen Reaktors in nennenswertem Umfang stattfindet.

Im erfindungsgemäßen Verfähren wird die zum Vorwärmen der Eduktströme notwendige Wärmeenergie zumindest teilweise, bevorzugt vollständig, aus dem Reaktoraustrag, d. h. aus dem heißen Produktstrom des erfindungsgemäßen Verfahrens, entnommen. In einer bevorzugten Ausführungsform des erfindungsgemäßen Verfahrens wird dazu in einem Wärmetauscher, beispielsweise einem Gegenstrom-Wärmetauscher, wenigstens ein Teil des Produktstromes mit wenigstens einem Teil, beispielsweise 70 bis 95%, von Gemisch G1 in Kontakt gebracht.

Die Temperatur des dem Reaktor zugeführten Stroms kann erfindungsgemäß über den Anteil des Gemischs G1, der über einen derartigen Wärmeaustauscher vorgewärmt wird, eingestellt werden.

Die vorliegende Erfindung betrifft somit ein Verfahren, wie oben beschrieben, dadurch gekennzeichnet, dass die zum Vorwärmen von Gemisch G1 und/oder G2 notwendige Wärmeenergie zumindest teilweise, bevorzugt vollständig, aus dem Produktstrom des erfindungsgemäßen Verfahrens, entnommen wird.

In einer bevorzugten Ausführungsform des erfindungsgemäßen Verfahrens wird wenigstens ein Teil des Produktstromes mit wenigstens einem Teil, beispielsweise 70 bis 95%, von Gemisch G1 in Kontakt gebracht, bevor der Produktstrom weiter aufgearbeitet wird.

Erfindungsgemäß weist der aus dem Verfahren erhaltene Produktstrom eine Reaktorausgangstemperatur (Tₐᵤₛ) von im Allgemeinen höchstens 340 °C, bevorzugt höchstens 320 °C, besonders bevorzugt höchstens 300 °C auf. Nach Inkontaktbringen mit dem Eduktstrom, bevorzugt mit Gemisch G1, weist der Produktstrom im Allgemeinen eine Temperatur von 150 bis 220 °C, bevorzugt 170 bis 200 °C, beispielsweise 180 bis 190 °C, auf. Der Eduktstrom, bevorzugt Gemisch G1, wird erfindungsgemäß auf im Allgemeinen 180 bis 280 °C, bevorzugt 240 bis 275 °C, beispielsweise 250 bis 260 °C, erwärmt. In einer besonders bevorzugten Ausführungsform des erfindungsgemäßen Verfahrens wird das auf eine Temperatur von im Allgemeinen 180 bis 280 °C, bevorzugt 240 bis 275 °C, beispielsweise 250 bis 260 °C vorgewärmte Gemisch G1 mit Gemisch G2 vermischt, so dass eine Temperatur des vereinigten Eduktstromes, bevorzugt enthaltend Gemisch G1 und G2, von bevorzugt 170 bis 270 °C, besonders bevorzugt 200 bis 260 °C, beispielsweise 220 bis 250 °C, resultiert.

Grundsätzlich kann erfindungsgemäß jedes Gemisch G1 enthaltend wenigstens ein monozyklisches Olefin mit 4 bis 20 Kohlenstoffatomen, bevorzugt mit 4 bis 8 Kohlenstoffatomen, eingesetzt werden.

Das in dem Gemisch G1 vorliegende wenigstens eine monozyklische Olefin mit 4 bis 20 Kohlenstoffatomen kann erfindungsgemäß eine oder mehrere Kohlenstoff-Kohlenstoff-Doppelbindung(en) aufweisen. In einer bevorzugten Ausführungsform weist das in Gemisch G1 vorliegende wenigstens eine monozyklische Olefin mit 4 bis 20 Kohlenstoffatomen eine Kohlenstoff-Kohlenstoff-Doppelbindung auf. Es ist erfindungsgemäß auch möglich, dass ein Gemisch G1 eingesetzt wird, welches neben wenigstens einem monozyklischen Olefin mit 4 bis 20 Kohlenstoffatomen mit einer Kohlenstoff-Kohlenstoff-Doppelbindung, ein oder mehrere monozyklische(s) Olefin(e) mit 4 bis 20 Kohlenstoffatomen mit zwei oder mehr Kohlenstoff-Kohlenstoff-Doppelbindungen enthält.

Besonders bevorzugt enthält das in dem erfindungsgemäßen Verfahren eingesetzte Gemisch G1 wenigstens ein monozyklisches Olefin ausgewählt aus der Gruppe bestehend aus Cyclobuten, Cyclopenten, Cyclohexen, Cyclohepten, Cycloocten, Cyclodecen, Cyclotetradecen, Cyclopentadecen, Cyclohexadecen, Cycloicosen und Mischungen davon. Ganz besonders bevorzugt enthält das in dem erfindungsgemäßen Verfahren eingesetzte Gemisch G1 ein monozyklisches Olefin ausgewählt aus der Gruppe bestehend aus Cyclopenten, Cyclohexen, Cyclohepten, Cycloocten und Mischungen davon. Cyclopenten (I), Cyclohexen (II), Cyclohepten (III) und Cycloocten (IV) sind im Folgenden abgebildet.

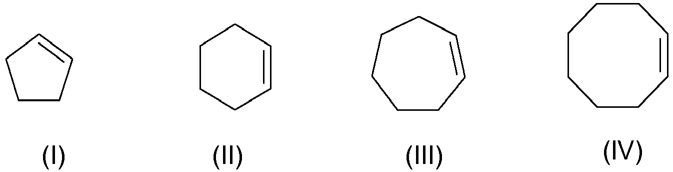

Grundsätzlich kann das Gemisch G1 zusätzlich zu Cyclopenten jede weitere Verbindung enthalten. Geeignet sind unter anderem auch Verbindungen, die ebenfalls mit Distickstoffmonoxid (N₂O) reagieren können. Bevorzugt sind hierbei solche Verbindungen, die zwar prinzipiell mit N₂O reagieren können, bei den erfindungsgemäß gewählten Reaktionsbedingungen jedoch gegenüber N₂O inert sind. Der Begriff "inert", wie er im Rahmen der vorliegenden Erfindung verwendet wird, bezeichnet Verbindungen, die bei den erfindungsgemäß gewählten Reaktionsbedingungen mit N₂O entweder nicht reagieren oder im Vergleich zur Reaktion von monozyklischen Olefinen mit 4 bis 20 Kohlenstoffatomen mit N₂O derart eingeschränkt reagieren, dass ihr Umsetzungsprodukt mit N₂O im resultierenden Gemisch zu höchstens 5 Gew.-%, bevorzugt zu höchstens 3 Gew.-% und besonders bevorzugt zu höchstens 2 Gew.-%, jeweils bezogen auf das Gesamtgewicht der resultierenden Mischung, enthalten ist.

In dem erfindungsgemäßen Verfahren kann in einer bevorzugten Ausführungsform ein Gemisch G1 eingesetzt werden, welches neben dem wenigstens einen Olefin mit 4 bis 20 Kohlenstoffatomen wenigstens einen weiteren Kohlenwasserstoff enthält.

Der Begriff "Kohlenwasserstoffe", wie er im Rahmen der vorliegenden Erfindung verwendet wird, bezeichnet Verbindungen, von denen jede ein nicht substituierter Kohlenwasserstoff ist und daher nur aus den Atomen C und H besteht, beispielsweise Olefine oder gesättigte Kohlenwasserstoffe.

In einer bevorzugten Ausführungsform enthält das eingesetzte Gemisch G1 neben dem wenigstens einen Olefin mit 4 bis 20 Kohlenstoffatomen wenigstens einen weiteren Kohlenwasserstoff mit 4 bis 20 Kohlenstoffatomen, beispielsweise ausgewählt aus der Gruppe bestehend aus Cyclopentan, Cyclohexan, Cycloheptan, 2-Buten, Isopentan, 1-Penten, 2-Methylbuten-1, *trans*-2-Penten, *n*-Pentan, *cis*-2-Penten, 2-Methylbuten-2, 2,2-Dimethylbutan, 2-Methylpentan, 3-Methylpentan, *n*-Hexan und Benzol. In einer besonders bevorzugten Ausführungsform enthält das in dem erfindungsgemäßen Verfahren eingesetzte Gemisch G1 neben einem Olefin ausgewählt aus der Gruppe bestehend aus Cyclopenten, Cyclohexen, Cyclohepten, Cycloocten und Mischungen davon, wenigstens einen gesättigten Kohlenwasserstoff, ausgewählt aus der Gruppe bestehend aus Cyclopentan, Cyclohexan, Cycloheptan, Cyclooctan und Mischungen davon.

Das wenigstens eine monozyklische Olefin mit 4 bis 20 Kohlenstoffatomen, insbesonders Cyclopenten, liegt dabei in dem Gemisch G1 im Allgemeinen in einer Menge von 20 bis 98 Gew.-%, bevorzugt 30 bis 80 Gew.-%, besonders bevorzugt 40 bis 60 Gew.-%, jeweils bezogen auf Gemisch G1, vor.

Der wenigstens eine weitere Kohlenwasserstoff, beispielsweise Cyclopentan, liegt in einer bevorzugten Ausführungsform in Gemisch G1 in einer Menge von 2 bis 80 Gew.-%, bevorzugt 20 bis 70 Gew.-%, besonders bevorzugt 40 bis 60 Gew.-%, jeweils bezogen auf Gemisch G1, vor.

In einer ganz besonders bevorzugten Ausführungsform enthält Gemisch G1 20 bis 98 Gew.-%, bevorzugt 30 bis 80 Gew.-%, besonders bevorzugt 40 bis 60 Gew.-%, jeweils bezogen auf Gemisch G1 wenigstens ein monozyklisches Olefin mit 4 bis 20 Kohlenstoffatomen, insbesondere Cyclopenten, und 2 bis 80 Gew.-%, bevorzugt 20 bis 70 Gew.-%, besonders bevorzugt 40 bis 60 Gew.-%, jeweils bezogen auf Gemisch G1, wenigstens einen weiteren Kohlenwasserstoff, insbesondere Cyclopentan. Demgemäß betrifft die vorliegende Erfindung auch ein Verfahren, wie oben beschrieben, dadurch gekennzeichnet, dass ein Gemisch G1 eingesetzt wird, enthaltend 20 bis 98 Gew.-%, bevorzugt 30 bis 80 Gew.-%, besonders bevorzugt 40 bis 60 Gew.-%, jeweils bezogen auf Gemisch G1 wenigstens ein monozyklisches Olefin mit 4 bis 20 Kohlenstoffatomen und 2 bis 80 Gew.-%, bevorzugt 20 bis 70 Gew.-%, besonders bevorzugt 40 bis 60 Gew.-%, jeweils bezogen auf Gemisch G1, wenigstens einen weiteren Kohlenwasserstoff.

Der Gehalt an anderen Komponenten im Gemisch G1 ist beispielsweise kleiner als 15 Gew.-%, vorzugsweise kleiner als 12 Gew.-%, bevorzugt kleiner als 10 Gew.-%, insbesondere kleiner als 8 Gew.-%, besonders bevorzugt kleiner als 5 Gew.-%.

Gemäß einer weiteren besonders bevorzugten Ausführungsform des erfindungsgemäßen Verfahrens besteht das Gemisch G1 zu mindestens 98 Gew.-%, bezogen auf das Gesamtgewicht des Gemisches G1, aus Kohlenwasserstoffen. Neben den Kohlenwasserstoffen kann das Gemisch G1 noch zu höchstens 5 Gew.-%, bevorzugt zu höchstens 2 Gew.-% mindestens eine weitere Verbindung enthalten, beispielsweise eine Verbindung ausgewählt aus der Gruppe bestehend aus Aldehyden, Ketonen, Epoxiden und Mischungen davon, beispielsweise Cyclopentanon, 3-Methyl-2-butanon, Epoxycyclopentan, 4-Pentenal, Aceton oder Mischungen davon. Diese Verbindungen können in der Reaktionsmischung unter der Maßgabe enthalten sein, dass sie die Umsetzung von wenigstens einem monozyklischen Olefin mit 4 bis 20 Kohlenstoffatomen mit dem Gemisch G2 nicht stören.

Gemäß einer bevorzugten Ausführungsform ist das Gemisch G1 bei den erfindungsgemäß gewählten Reaktionsbedingungen gasförmig, flüssig oder überkritisch, bevorzugt überkritisch.

Im Rahmen einer ebenfalls bevorzugten Ausführungsform des erfindungsgemäßen Verfahrens wird ein Gemisch G1 eingesetzt, das zu mindestens 90 Gew.-%, bevorzugt zu mindestens 95 Gew.-%, insbesondere zu mindestens 98 Gew.-% aus C₅-Kohlenwasserstoffen und Kohlenwasserstoffen mit mehr als 5 Kohlenstoffatomen besteht. Neben Cyclopenten können demgemäß mindestens ein weiterer C₅-Kohlenwasserstoff, beispielsweise *n*-Pentan und/oder Cyclopentan, oder mindestens ein Kohlenwasserstoff mit mehr als 5 Kohlenstoffatomen, beispielsweise Cyclohexan, oder ein Gemisch aus mindestens einem weiteren C₅-Kohlenwasserstoff und mindestens einem Kohlenwasserstoff mit mehr als 5 Kohlenstoffatomen in G1 enthalten sein.

Demgemäß beschreibt die vorliegende Erfindung auch ein Verfahren, wie oben beschrieben, das dadurch gekennzeichnet ist, dass das Gemisch G1 mindestens 98 Gew.-% C₅-Kohlenwasserstoffe und Kohlenwasserstoffe mit mehr als 5 Kohlenstoffatomen enthält.

Als unter anderem besonders bevorzugte Kohlenwasserstoffe mit mehr als 5 Kohlenstoffatomen werden die entsprechenden, bereits oben im Rahmen der weiteren Kohlenwasserstoffe genannten Kohlenwasserstoffe eingesetzt.

Erfindungsgemäß werden als Gemisch G1 bevorzugt solche Gemische eingesetzt, die in großtechnischen Verfahren anfallen. Im Rahmen der vorliegenden Erfindung sind hierbei Gemische bevorzugt, die zu mindestens 95 Gew.-%, weiter bevorzugt zu mindestens 96 Gew.-% und besonders bevorzugt zu mindestens 97 Gew.-% aus C₅- und C₆- oder C₅- und C₇- oder C₅- und C₆- und C₇-Kohlenwasserstoffen bestehen.

Demgemäß betrifft die vorliegende Erfindung auch ein Verfahren, wie oben beschrieben, das dadurch gekennzeichnet ist, dass das Gemisch G1 zu mindestens 95 Gew.-% aus C₅- und C₆- oder C₅- und C₇- oder C₅- und C₆- und C₇-Kohlenwasserstoffen besteht.

Im Rahmen der vorliegenden Erfindung kann dabei das Gemisch G1 neben Cyclopenten entweder mindestens einen weiteren C₅-Kohlenwasserstoff oder mindestens einen C₆-Kohlenwasserstoff oder mindestens einen C₇-Kohlenwasserstoff oder ein Gemisch aus mindestens einem weiteren C₅-Kohlenwasserstoff und mindestens einem C₆-Kohlenwasserstoff oder ein Gemisch aus mindestens einem weiteren C₅-Kohlenwasserstoff und mindestens einem C₇-Kohlenwasserstoff oder ein Gemisch aus mindestens einem weiteren C₅-Kohlenwasserstoff und mindestens einem C₆-Kohlenwasserstoff und mindestens einem C₇-Kohlenwasserstoff enthalten.

Erfindungsgemäß kann das eingesetzte Gemisch G1 aus jeder beliebigen Quelle stammen. Bevorzugt stammt im Rahmen der vorliegenden Erfindung das Gemisch G1 zumindest teilweise aus nicht umgesetztem und rückgeführtem Edukt des Verfahrens.

Erfindungsgemäß stammt das Gemisch G1 zumindest teilweise aus einer anderen Quelle. Im Rahmen einer bevorzugten Ausführungsform des erfindungsgemäßen Verfahrens wird als Gemisch G1 zumindest teilweise ein Kohlenwasserstoffgemisch eingesetzt, das aus einem Steamcracker oder einer Raffinerie gewonnen wird und Cyclopenten enthält. In diesem Zusammenhang sind beispielsweise C₅-Schnitte aus Steamcracker-Anlagen bevorzugt, die im Wesentlichen nur C₅- und C₆-Kohlenwasserstoffe enthalten. Kohlenwasserstoffe mit mehr als 6 Kohlenstoffatomen sind in den großtechnisch anfallenden C₅-Schnitten üblicherweise nicht enthalten. Diese großtechnisch anfallenden C₅-Schnitte umfassen neben Cyclopenten beispielsweise 2-Buten, Isopentan, 1-Penten, 2-Methylbuten-1, *trans*-2-Penten, *n*-Pentan, *cis*-2-Penten, 2-Methylbuten-2, Cyclopentan, 2,2-Dimethylbutan, 2-Methylpentan, 3-Methylpentan, *n*-Hexan und Benzol. Im Allgemeinen enthält ein C₅-Schnitt aus einer Steamcracker-Anlage Cyclopenten im Bereich von 5 bis 60 Gew.-% und bevorzugt im Bereich von 15 bis 50 Gew.-%. Derartige Gemische werden vorteilhafterweise weiter aufgereinigt, bevor sie als Gemisch G1 in das erfindungsgemäße Verfahren eingesetzt werden.

Daher beschreibt die vorliegende Erfindung auch ein Verfahren, wie oben beschrieben, das dadurch gekennzeichnet ist, dass das Gemisch G1 zu mindestens 95 Gew.-% ein Gemisch aus C₅- und C₆-Kohlenwasserstoffen enthält.

Erfindungsgemäß kann dieses Gemisch aus im Wesentlichen C₅- und C₆-Kohlenwasserstoffen, das bevorzugt als C₅-Schnitt aus einer Steamcracker-Anlage oder das aus der Partialhydrierung von Cyclopentadien gewonnen wird, als solches eingesetzt werden. Bevorzugt wird das Gemisch aus im wesentlichen C₅- und C₆-Kohlenwasserstoffen vor der erfindungsgemäßen Umsetzung einer Reinigung unterzogen, bei der wiederum bevorzugt im Vergleich zu Cyclopenten leichter siedende Verbindungen abgetrennt werden. Während hierbei sämtliche denkbaren Methoden einsetzbar sind, ist die destillative Auftrennung des Gemisches bevorzugt.

Insbesondere betrifft die vorliegende Erfindung daher auch ein Verfahren, wie oben beschrieben, dadurch gekennzeichnet, dass ein cyclopentenhaltiges Kohlenwasserstoffgemisch als Edukt zur Herstellung von Cyclopentanon eingesetzt wird, wobei das cyclopentenhaltige Kohlenwasserstoffgemisch entweder der C₅-Schnitt einer Steamcracker-Anlage ist oder aus der Partialhydrierung von Cyclopentadien erhalten wird.

Durch das erfindungsgemäße Verfahren werden durch Umsetzung von monozyklischen Olefinen mit 4 bis 20 Kohlenstoffatome mit Distickstoffmonoxid die entsprechenden monozyklischen Ketone mit 4 bis 20 Kohlenstoffatomen erhalten. Werden Olefine eingesetzt, die eine Kohlenstoff-Kohlenstoff-Doppelbindungen enthalten, werden durch das erfindungsgemäße Verfahren Monoketone erhalten. Werden Olefine eingesetzt, die zwei oder mehr Kohlenstoff-Kohlenstoff-Doppelbindungen enthalten, so ist es im Rahmen der vorliegenden Erfindung möglich, dass nur eine der Kohlenstoff-Kohlenstoff-Doppelbindungen reagiert. Ebenso ist es jedoch auch möglich, dass zwei oder mehr der Kohlenstoff-Kohlenstoff-Doppelbindungen reagieren. In diesem Fall werden durch das erfindungsgemäße Verfahren entsprechende Ketone erhalten, die eine, zwei oder mehrere Keton-Funktionalitäten enthalten.

Für den erfindungsgemäß besonders bevorzugten Fall, dass das in dem erfindungsgemäßen Verfahren eingesetzte Gemisch G1 ein monozyklisches Olefin ausgewählt aus der Gruppe bestehend aus Cyclopenten, Cyclohexen, Cyclohepten, Cycloocten und Mischungen davon, enthält, wird ein monozyklische Keton ausgewählt aus der Gruppe bestehend aus Cyclopentanon, Cyclohexanon, Cycloheptanon, Cyclooctanon und Mischungen davon erhalten. Cyclopentanon (V), Cyclohexanon (VI), Cycloheptanon (VII) und Cyclooctanon (VIII) sind im Folgenden abgebildet.

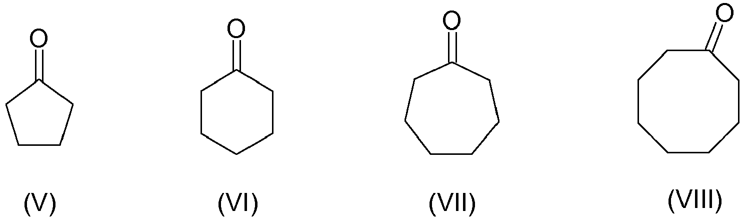

In einer ganz besonders bevorzugten Ausführungsform dient das erfindungsgemäße Verfahren zur Herstellung von Cyclopentanon aus Cyclopenten und Distickstoffmonoxid.

In dem erfindungsgemäßen Verfahren wird im Allgemeinen ein Gemisch G2 enthaltend wenigstens Distickstoffmonoxid eingesetzt.

Das Gemisch G2 enthält erfindungsgemäß mindestens 70 Vol.-% Distickstoffmonoxid, beispielsweise 70 bis 100 Vol.-%. Vorzugsweise enthält das Gemisch G2 mindestens 75 Vol.-% Distickstoffmonoxid, insbesondere mindestens 80 Vol.-%, bevorzugt mindestens 85 Vol.-%. Das Gemisch G2 enthält bevorzugt von 75 bis 99 Vol.-% Distickstoffmonoxid, besonders bevorzugt von 80 bis 95 Vol.-%, insbesondere bevorzugt von 82 bis 90 Vol.-%, beispielsweise 83 Vol.-%, 84 Vol.-%, 85 Vol.-%, 86 Vol.-%, 87 Vol.-%, 88 Vol.-% oder 89 Vol.-%.

Daher betrifft die vorliegende Erfindung gemäß einer weiteren Ausführungsform auch das oben beschriebene Verfahren zur Herstellung von wenigstens einem monozyklischen Keton mit 4 bis 20 Kohlenstoffatomen, umfassend die Umsetzung eines Gemischs G1 enthaltend wenigstens ein monozyklisches Olefin mit 4 bis 20 Kohlenstoffatomen mit einem Gemisch G2 enthaltend wenigstens Distickstoffmonoxid, wobei diese Umsetzung adiabatisch durchgeführt wird und das Gemisch G2 mindestens 70 Vol.-% Distickstoffmonoxid enthält.

Die vorliegende Erfindung betrifft gemäß einer weiteren Ausführungsform auch das oben beschriebene Verfahren zur Herstellung von wenigstens einem monozyklischen Keton mit 4 bis 20 Kohlenstoffatomen, umfassend die Umsetzung eines Gemischs G1 enthaltend wenigstens ein monozyklisches Olefin mit 4 bis 20 Kohlenstoffatomen mit einem Gemisch G2 enthaltend wenigstens Distickstoffmonoxid, wobei diese Umsetzung adiabatisch durchgeführt wird und das Gemisch G2 75 bis 99 Vol.-% Distickstoffmonoxid enthält.

Grundsätzlich kann das Gemisch G2 enthaltend Distickstoffmonoxid aus jeder beliebigen Quelle stammen.

Erfindungsgemäß wird dieses Gemisch G2 vorzugsweise verflüssigt und dann in flüssiger Form eingesetzt. Dabei kann Distickstoffmonoxid oder das Gasgemisch enthaltend Distickstoffmonoxid mit allen dem Fachmann bekannten Verfahren verflüssigt werden, insbesondere durch geeignete Wahl des Drucks und der Temperatur.

Gemisch G2 kann erfindungsgemäß neben Distickstoffmonoxid noch mindestens ein weiteres Gas enthalten. Hierbei sind im Wesentlichen sämtliche Gase denkbar, solange gewährleistet ist, dass die erfindungsgemäße Umsetzung von wenigstens einem monozyklischen Olefin mit 4 bis 20 Kohlenstoffatomen mit Distickstoffmonoxid möglich ist. Insbesondere sind demzufolge Gemische G2 bevorzugt, die neben Distickstoffmonoxid mindestens ein Inertgas enthalten. Der Begriff "Inertgas", wie er im Rahmen der vorliegenden Erfindung verwendet wird, bezeichnet ein Gas, das sich hinsichtlich der Umsetzung von Distickstoffmonoxid mit monozyklischen Olefinen mit 4 bis 20 Kohlenstoffatomen als auch gegenüber Distickstoffmonoxid unter den Reaktionsbedingungen inert verhält. Als Inertgase sind beispielsweise Stickstoff, Kohlendioxid, Kohlenmonoxid, Argon, Methan, Ethan und Propan zu nennen.

Ebenso können im Gemisch G2 auch Gase enthalten sein, die sich bei der Umsetzung von Distickstoffmonoxid mit monozyklischen Olefinen mit 4 bis 20 Kohlenstoffatomen nicht als Inertgase verhalten. Als solche Gase sind unter anderem NOₓ oder beispielsweise Sauerstoff zu nennen. Der Begriff "NOₓ", wie er im Rahmen der vorliegenden Erfindung verstanden wird, bezeichnet sämtliche Verbindungen NₐO_{b} außer Distickstoffmonoxid (N₂O), wobei a 1 oder 2 ist und b eine Zahl von 1 bis 6. Statt dem Begriff "NOₓ" wird im Rahmen der vorliegenden Erfindung auch der Begriff "Stickoxide" verwendet. In einem solchen Fall ist es bevorzugt, solche Gemische G2 einzusetzen, deren Gehalt an diesen Gasen höchstens 0,5 Vol.-%, bezogen auf das Gesamtgewicht des Gemisches G2, beträgt.

Demgemäß betrifft die vorliegende Erfindung auch das oben beschriebene Verfahren zur Herstellung von wenigstens einem monozyklischen Keton mit 4 bis 20 Kohlenstoffatomen, umfassend die Umsetzung eines Gemischs G1 enthaltend wenigstens ein monozyklisches Olefin mit 4 bis 20 Kohlenstoffatomen mit einem Gemisch G2 enthaltend wenigstens Distickstoffmonoxid, wobei das Gemisch G2 höchstens 0,5 Vol.-% Sauerstoff oder höchstens 0,5 Vol.-% Stickoxide oder höchstens sowohl 0,5 Vol.-% Sauerstoff als auch 0,5 Vol.-% Stickoxide, jeweils bezogen auf das Gesamtvolumen des Gemisches G2, enthält. Ein Wert von beispielsweise 0,5 Vol.-% bezeichnet hierbei einen Gesamtgehalt aller möglichen Stickoxide außer Distickstoffmonoxid von 0,5 Vol.-%.

Grundsätzlich kann die Zusammensetzung der Gemische im Rahmen der vorliegenden Erfindung auf jede dem Fachmann bekannte Weise bestimmt werden. Die Zusammensetzung des Gemischs G2 wird im Rahmen der vorliegenden Erfindung vorzugsweise gaschromatographisch bestimmt. Sie kann jedoch auch mittels UV-Spektroskopie, IR-Spektroskopie oder nasschemisch bestimmt werden.

Erfindungsgemäß wird das Gemisch G2 insbesondere in flüssiger oder überkritischer Form eingesetzt. Dabei ist es erfindungsgemäß möglich, dass das Gemisch G2 vor der Verflüssigung einer Behandlung unterzogen wird, um die Konzentration von inerten und störenden Verbindungen im Gemisch G2 zu reduzieren.

Insbesondere ist es im Rahmen der vorliegenden Erfindung möglich, Gemische G2 einzusetzen, die aus großtechnischen Verfahren erhalten werden. Sollten demgemäß diese Gemische G2 mehr als 0,5 Vol.-% Sauerstoff und/oder Stickoxide enthalten, so können diese im Allgemeinen im erfindungsgemäßen Verfahren eingesetzt werden. Bevorzugt werden diese Gemische G2, wie auch solche Gemische G2 ähnlicher Zusammensetzung, die nicht aus großtechnischen Verfahren erhalten werden, vor dem Einsatz im erfindungsgemäßen Verfahren mindestens einem Reinigungsschritt unterworfen, in dem der Gehalt an Sauerstoff und/oder Stickoxiden auf höchstens 0,5 Vol.-% eingestellt wird.

Ein im Rahmen der vorliegenden Erfindung geeignetes Gasgemisch G2 enthält vorzugsweise 50 bis 99,0 Vol.-% Distickstoffmonoxid, 1 bis 20 Vol.-% Kohlenstoffdioxid und 0 bis 25 Vol.-% weitere Gase. Die angegebenen Vol.-% beziehen sich jeweils auf das gesamte Gasgemisch G2. Die Summe der einzelnen Komponenten des Gasgemischs G2 ergibt dabei 100 Vol.-%.

Vorzugsweise enthält das Gasgemisch G2 60 bis 95 Vol.-% Distickstoffmonoxid, insbesondere 70 bis 90 Vol.-%, besonders bevorzugt 75 bis 89 Vol.-% Distickstoffmonoxid.

Das Gasgemisch G2 kann weiterhin 1 bis 20 Vol.-% Kohlenstoffdioxid enthalten. Vorzugsweise enthält das Gasgemisch G2 5 bis 15 Vol.-% Kohlenstoffdioxid, insbesondere 6 bis 14 Vol.-% Kohlenstoffdioxid.

Vorzugsweise enthält das Gasgemisch G2 0 bis 25 Vol.-% weitere Gase. Das Gasgemisch G2 kann ein oder mehrere weitere Gase enthalten, wobei die angegebene Menge auf die Summe der enthaltenen Gase bezogen ist.

Geeignete Verfahren zur Herstellung eines derartigen Gasgemischs sind dem Fachmann an sich bekannt.

Gemäß dem erfindungsgemäßen Verfahren wird als Produktstrom bevorzugt ein Reaktionsgemisch G3 erhalten, welches das wenigstens eine monozyklische Keton mit 4 bis 20 Kohlenstoffatomen, bevorzugt Cyclopentanon und Stickstoff, enthält. Neben diesen gewünschten Produkten liegen in dem Gemisch G3 beispielsweise nicht umgesetzte Edukte und/oder Nebenprodukte vor.

Das mit dem erfindungsgemäßen Verfahren erhaltene wenigstens eine monozyklische Keton mit 4 bis 20 Kohlenstoffatomen, bevorzugt Cyclopentanon, bzw. das nach dem erfindungsgemäßen Verfahren erhaltene Reaktionsgemisch G3 enthaltend das wenigstens eine monozyklische Keton mit 4 bis 20 Kohlenstoffatomen, bevorzugt Cyclopentanon, kann prinzipiell in der erhaltenen Form weiter verarbeitet werden. Erfindungsgemäß kann das erhaltene Gemisch G3 jedoch auch gemäß sämtlicher geeigneter Verfahren zur Gewinnung des wenigstens einen monozyklischen Ketons mit 4 bis 20 Kohlenstoffatomen, bevorzugt Cyclopentanon, aufgearbeitet werden. Besonders bevorzugt sind erfindungsgemäß destillative Verfahren zur Aufarbeitung. Bevorzugt erfolgt im Rahmen der vorliegenden Erfindung eine weitere Aufarbeitung in einer Trennstufe (B).

Somit betrifft die vorliegende Erfindung in einer bevorzugten Ausführungsform ein Verfahren, wie oben beschrieben, wenigstens umfassend die folgenden Schritte:
(A) Umsetzung eines Gemischs G1 enthaltend wenigstens ein monozyklisches Olefin mit 4 bis 20 Kohlenstoffatomen mit einem Gemisch G2 enthaltend wenigstens Distickstoffmonoxid unter adiabatischen Bedingungen, um ein Reaktionsgemisch G3 zu erhalten und
(B) Abtrennen des wenigstens einen monozyklischen Ketons mit 4 bis 20 Kohlenstoffatomen aus dem in Schritt (A) erhaltenen Reaktionsgemisch G3.

Stufe (A) des erfindungsgemäß bevorzugten Verfahrens und die bevorzugten Ausführungsformen sind bereits oben detailliert beschrieben worden.

Stufe (B) des erfindungsgemäß bevorzugten Verfahrens zur Herstellung von wenigstens einem monozyklischen Keton mit 4 bis 20 Kohlenstoffatomen umfasst das Abtrennen des wenigstens einen monozyklischen Ketons mit 4 bis 20 Kohlenstoffatomen aus dem in Schritt (A) erhaltenen Reaktionsgemisch G3.

Dabei kann erfindungsgemäß die Trennstufe (B) einen oder mehrere Reinigungsschritte umfassen.

Die Trennstufe (B) umfasst vorzugsweise mindestens eine Destillation, bevorzugt aber mindestens eine einstufige Verdampfung, beispielsweise zur Abtrennung von N₂ und nicht umgesetztem Distickstoffmonoxid, und mindestens eine Destillation, besonders bevorzugt mindestens eine einstufige Verdampfung und mindestens zwei Destillationsschritte.

In einer bevorzugten Ausführungsform des erfindungsgemäßen Verfahrens wird das Gemisch G3 in der Trennstufe (B) zunächst in wenigstens einem geeigneten Behälter B1 auf einen Druck entspannt, der im Allgemeinen unterhalb des Reaktionsdruckes liegt, beispielsweise auf einen Druck von 1 bis 20 bar, bevorzugt 14 bis 18 bar. In einer bevorzugten Ausführungsform wird Gemisch G3 vor diesem Entspannen in einem geeigneten Wärmetauscher abgekühlt.

In einer ganz besonders bevorzugten Ausführungsform des erfindungsgemäßen Verfahrens wird mit dem direkt aus der erfindungsgemäßen Umsetzung erhaltenen Gemisch G3 zunächst wenigstens ein Eduktstrom, wie oben beschrieben, vorgewärmt, und anschließend wird Gemisch G3 auf den genannten Druck entspannt.

Gemäß einer bevorzugten Ausführungsform der vorliegenden Erfindung umfasst die Trennstufe (B) eine einstufige Verdampfung in einem Behälter B1 und mindestens einen Destillationsschritt in einer geeigneten Destillationskolonne, vorzugsweise in einer Rückführkolonne K1. Gemäß einer derartigen Ausführungsform wird nach der Entspannung des Gemischs G3 in Behälter B1 ein flüssiges Gemisch (G3f) und ein gasförmiges Gemisch (G3g) erhalten.

Das Gemisch G3g kann erfindungsgemäß bevorzugt mit einem oder mehreren, bevorzugt zwei, Wärmetauschern abgekühlt werden, beispielsweise auf eine Temperatur von maximal 50°C, bevorzugt auf eine Temperatur unterhalb von 5°C und besonders bevorzugt auf eine Temperatur unterhalb -10°C. Die Wärmetauscher können mit allen dem Fachmann bekannten Kühlmedien betrieben werden, beispielsweise Wasser, Sole, etc. In einer bevorzugte Ausführungsform des Verfahrens wird das Gemisch G3g in einem ersten Wärmetauscher mittels Kühlwasser auf eine Temperatur von maximal 50°C abgekühlt und in einem zweiten Wärmetauscher mittels Sole auf eine Temperatur von maximal 5°C, bevorzugt von maximal -10°C abgekühlt. Bei der Abkühlung kondensiert jeweils einen Anteil des gasförmigen Gemisches. Bevorzugt wird dieser kondensierte Anteil wieder in den Behälter B1 zurückgeführt und mit dem Gemisch G3f unter Erhalt eines Gemischs G3f' vereinigt. Dieses Vorgehen hat den Vorteil, dass organische Komponenten, die sich im Gemisch G3g befinden, weiter umgesetzt werden können. Das erhaltene gasförmige Gemisch G3g bzw. das nach Durchlaufen der Wärmetauscher erhaltene gasförmige Gemisch kann nach dem Fachmann bekannten Verfahren abgetrennt werden.

Das erhaltene flüssige Gemisch G3f bzw. das Gemisch G3f' wird gemäß einer bevorzugten Ausführungsform auf einen Druck von 1 bis 5 bar, beispielsweise 3 bar entspannt und wenigstens einer Destillation unterworfen, um die in Gemisch G3f bzw. G3f' vorliegenden Komponenten voneinander zu trennen.

Die in Trennstufe (B) bevorzugt durchgeführte Destillation kann nach allen dem Fachmann bekannten Verfahren erfolgen. Temperaturen, Druck, Ausgestaltung der Destillationskolonne(n) etc. richten sich dabei nach den zu trennenden Stoffen.

In Trennstufe (B) des erfindungsgemäßen Verfahrens wird bevorzugt das gewünschte Produkt, wenigstens ein Keton mit 4 bis 20 Kohlenstoffatomen, von nicht umgesetztem Edukt, ggf. weiteren in der Reaktionsmischung vorliegenden Komponenten und gegebenenfalls gebildeten Nebenprodukten getrennt. In einer besonders bevorzugten Ausführungsform von Trennstufe (B) wird das Produkt Cyclopentanon von nicht umgesetztem Cyclopenten und gegebenenfalls in der Reaktionsmischung vorliegendem Cyclopentan getrennt.

In einer besonders bevorzugten Ausführungsform des erfindungsgemäßen Verfahrens wird zur Destillation des Gemischs G3f bzw. G3f' in Trennstufe (B) eine Rückführkolonne K1 eingesetzt, die im Allgemeinen 30 bis 50, bevorzugt 35 bis 45, theoretische Böden aufweist. Der Zulauf erfolgt im Allgemeinen im mittleren Teil der Kolonne. In einer weiteren bevorzugten Ausführungsform wird beispielsweise im oberen Teil der in Trennstufe (B) eingesetzten Rückführkolonne K1 nicht umgesetztes monozyklisches Olefin mit 4 bis 20 Kohlenstoffatomen gewonnen. Besonders bevorzugt wird Cyclopenten in einem Seitenabzug der Kolonne K1 gewonnen.

Ein geeigneter Strom enthaltend abdestilliertes nicht umgesetztes monozyklisches Olefin mit 4 bis 20 Kohlenstoffatomen, besonders bevorzugt Cyclopenten, kann dann beispielsweise zurückgeführt werden und alleine oder nach Zusatz eines geeigneten Gemischs enthaltend monozyklisches Olefin mit 4 bis 20 Kohlenstoffatomen, besonders bevorzugt Cyclopenten, als Gemisch G1 im erfindungsgemäßen Verfahren eingesetzt werden.

In einer besonders bevorzugten Ausführungsform wird das in der Trennstufe (B), zum Beispiel im Seitenabzug der Kolonne K1 abgetrennte nicht umgesetzte monozyklische Olefin mit 4 bis 20 Kohlenstoffatomen in Stufe (A) des erfindungsgemäßen Verfahrens, d. h. in Gemisch G1, zurückgeführt.

Somit betrifft die vorliegende Erfindung in einer bevorzugten Ausführungsform das oben beschriebene Verfahren, dadurch gekennzeichnet, dass in der Trennstufe (B) nicht umgesetztes monozyklisches Olefin mit 4 bis 20 Kohlenstoffatomen abgetrennt wird.

Somit betrifft die vorliegende Erfindung in einer weiteren bevorzugten Ausführungsform das oben beschriebene Verfahren, dadurch gekennzeichnet, dass das in der Trennstufe (B) abgetrennte nicht umgesetzte monozyklische Olefin mit 4 bis 20 Kohlenstoffatomen in Stufe (A) des Verfahrens zurückgeführt wird.

Die Destillation in Trennstufe (B) in der Rückführkolonne K1 erfolgt beispielsweise bei einem Druck von 1,0 bis 7,0 bar, bevorzugt 2,0 bis 6,0 bar, beispielsweise 3,5 bis 5,0 bar.

Die Destillation in Trennstufe (B) in der Rückführkolonne K1 erfolgt beispielsweise bei einer Temperatur von 80 bis 200 °C, bevorzugt 90 bis 190 °C. Dabei beträgt die Temperatur im Sumpf der Kolonne beispielsweise 150 bis 200 °C, bevorzugt 160 bis 185 °C, die Temperatur oberhalb des Sumpfes der Kolonne beträgt beispielsweise 80 bis 110 °C, bevorzugt 90 bis 105 °C.

Das wenigstens eine nicht umgesetzte monozyklische Olefin mit 4 bis 20 Kohlenstoffatomen kann erfindungsgemäß in unterschiedlicher Reinheit erhalten werden. Das wenigstens eine nicht umgesetzte monozyklische Olefin mit 4 bis 20 Kohlenstoffatomen kann erfindungsgemäß beispielsweise in reiner Form, d. h. mit einem Gehalt von größer 90 Gew.-%, bevorzugt 95 Gew.-%, erhalten werden.

In einer weiteren Ausführungsform der Trennstufe (B) wird wenigstens ein nicht umgesetzte monozyklische Olefin mit 4 bis 20 Kohlenstoffatomen in Mischung mit weiteren Kohlenwasserstoffen, beispielsweise gesättigten Kohlenwasserstoffen, beispielsweise Cyclopentan, erhalten, beispielsweise als eine Mischung enthaltend 20 bis 98 Gew.-%, bevorzugt 30 bis 80 Gew.-%, besonders bevorzugt 40 bis 60 Gew.-%, jeweils bezogen auf die Mischung wenigstens ein Olefin mit 4 bis 20 Kohlenstoffatomen, insbesondere Cyclopenten, und 2 bis 80 Gew.-%, bevorzugt 20 bis 70 Gew.-%, besonders bevorzugt 40 bis 60 Gew.-%, jeweils bezogen auf die Mischung, wenigstens einen weiteren Kohlenwasserstoff, beispielsweise einen gesättigten Kohlenwasserstoff, insbesondere Cyclopentan. Diese Mischung kann weitere Komponenten, beispielsweise Kohlenwasserstoffe, Produkt bzw. Nebenprodukt aus Stufe (A) und/oder lineare Olefine, bis zu einem Gesamtgehalt von bis zu 1,5 Gew.-%, bevorzugt bis zu 1,0 Gew.-%, jeweils bezogen auf die Mischung, enthalten.

In einer weiteren bevorzugten Ausführungsform der Trennstufe (B) werden am Kopf der Rückführkolonne K1 niedrig siedende Komponenten erhalten, beispielsweise C₅-Kohlenwasserstoffe wie *n*-Pentan, 2-Methyl-2-buten, *cis*-2-Penten, und *trans*-2-Penten.

Das gewünschte Produkt, also das wenigstens eine monozyklische Keton mit 4 bis 20 Kohlenstoffatomen, insbesondere Cyclopentanon, wird in einer weiteren bevorzugten Ausführungsform der Trennstufe (B) im Sumpf der Rückführkolonne K1 erhalten, in einer bevorzugten Ausführungsform mit einer Reinheit von bis zu 95 Gew.-%, bevorzugt bis zu 92 Gew.-%, jeweils bezogen auf die Sumpffraktion.

Erfindungsgemäß kann die Trennstufe (B) auch so ausgestaltet sein, dass das wenigstens eine monozyklische Keton mit 4 bis 20 Kohlenstoffatomen, insbesondere Cyclopentanon, in geringerer Reinheit erhalten wird.

Erfindungsgemäß ist es auch möglich, dass die Trennstufe (B) neben der einstufigen Verdampfung und der ersten Destillation, vorzugsweise der Destillation in einer Rückführkolonne K1, eine weitere Destillation umfasst. So ist es erfindungsgemäß möglich, dass zur weiteren Aufreinigung des Produktes das wenigstens eine monozyklische Keton mit 4 bis 20 Kohlenstoffatomen, insbesondere Cyclopentanon, destilliert werden kann, beispielsweise in einer oder mehreren Kolonnen, bevorzugt in zwei Kolonnen oder besonders bevorzugt in einer Trennwandkolonne K2.

Erfindungsgemäß umfasst die Trennstufe (B) gemäß einer bevorzugten Ausführungsform eine einstufige Verdampfung in einem Behälter B1, eine Destillation in einer Rückführkolonne K1 und eine weitere Destillation in einer Destillationskolonne K2, beispielsweise in einer Trennwandkolonne.

Die Aufreinigung des erfindungsgemäß aus der Destillation in der Rückführkolonne K1 erhaltenen Produktes erfolgt beispielsweise bei einem Druck von 0,5 bis 3 bar, bevorzugt 0,8 bis 2 bar, beispielsweise 1,0 bis 1,2 bar.

Die Aufreinigung des erfindungsgemäß aus der Destillation in der Rückführkolonne K1 erhaltenen Produktes erfolgt beispielsweise bei einer Temperatur von 100 bis 200 °C, bevorzugt 110 bis 180 °C, beispielsweise 120 bis 170 °C.

Beispielsweise erfolgt die Aufreinigung des erfindungsgemäß aus der Destillation in der Rückführkolonne K1 erhaltenen Produktes beispielsweise in einer Trennwandkolonne K2 bei einem Druck von 0,5 bis 3 bar, bevorzugt 0,8 bis 2 bar, beispielsweise 1,0 bis 1,2 bar und bei einer Temperatur von 100 bis 200 °C, bevorzugt 110 bis 180 °C, beispielsweise 120 bis 170 °C

Die vorliegende Erfindung betrifft auch Verfahren umfassend wenigstens die Schritte (A) und (B), wie oben beschrieben, dadurch gekennzeichnet, dass das Gemisch G1 und/oder G2 vor der Umsetzung zu wenigstens einem monozyklischen Keton mit 4 bis 20 Kohlenstoffatomen in Schritt (A) auf eine Temperatur von 170 bis 270 °C vorgewärmt werden. Weitere Details und bevorzugte Ausführungsformen sind oben bereits angegeben.

Somit betrifft die vorliegende Erfindung in einer bevorzugten Ausführungsform ein Verfahren, wie oben beschrieben, wenigstens umfassend die folgenden Schritte:
(A) Umsetzung eines Gemischs G1 wie oben definiert, enthaltend wenigstens ein monozyklisches Olefin mit 4 bis 20 Kohlenstoffatomen mit einem Gemisch G2 enthaltend wenigstens Distickstoffmonoxid unter adiabatischen Bedingungen, um ein Reaktionsgemisch G3 zu erhalten und
(B) Abtrennen des wenigstens einen monozyklischen Ketons mit 4 bis 20 Kohlenstoffatomen aus dem in Schritt (A) erhaltenen Reaktionsgemisch G3,
dadurch gekennzeichnet, dass das Gemisch G1 und/oder G2 vor der Umsetzung zu wenigstens einem monozyklischen Keton mit 4 bis 20 Kohlenstoffatomen in Schritt (A) auf eine Temperatur von 170 bis 270 °C vorgewärmt werden.

In einer bevorzugten Ausführungsform dieses Verfahrens wird die für das Vorwärmen notwendige Wärmeenergie zumindest teilweise, bevorzugt vollständig, aus dem Produktstrom des erfindungsgemäßen Verfahrens, entnommen.

Das erfindungsgemäße Verfahren ist besonders vorteilhaft für die Oxidation von zyklischen Olefinen mit Distickstoffmonoxid. Ebenso eignet sich das Verfahren jedoch grundsätzlich auch für die Oxidation anderer Verbindungen mittels Distickstoffmonoxid, insbesondere von Verbindungen, die eine CC-Doppelbindung enthalten, wie z.B. nicht zyklische Olefine oder substituierten Olefine, wie z.B. Enolether.

Im folgenden wird die Erfindung durch Figuren und Beispiele näher erläutert.

### Figuren:

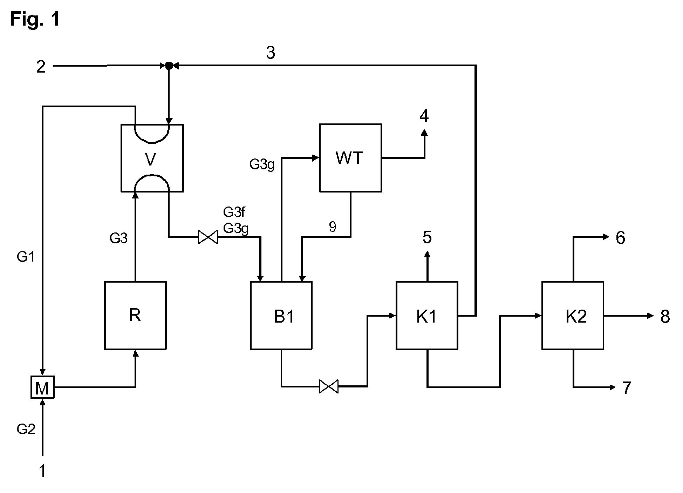

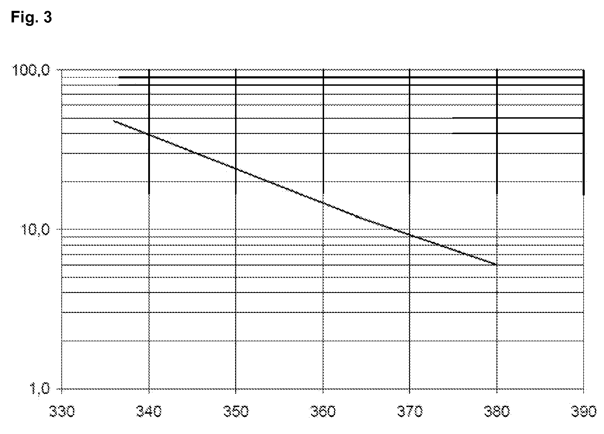

- Figur 1: zeigt eine Skizze einer Versuchsanlage, in der das erfindungsgemäße Verfahren durchgeführt werden kann. Die Bezugszeichen haben die folgenden Bedeutungen:
1 Distickstoffmonoxid-Feed
2 Cyclopenten-Feed
3 Rückgeführtes, nicht umgesetztes Cyclopenten
4 Abgasstrom
5 Leichtsieder purge
6 Leichtsieder
7 Schwersieder
8 Produkt Cyclopentanon
9 Kondensat
M Mischer
R Reaktor
WT Wärmetauscher/Partialkondensator
B1 Phasentrennbehälter
K1 Rückführkolonne
K2 Feinreinigungskolonne(n)

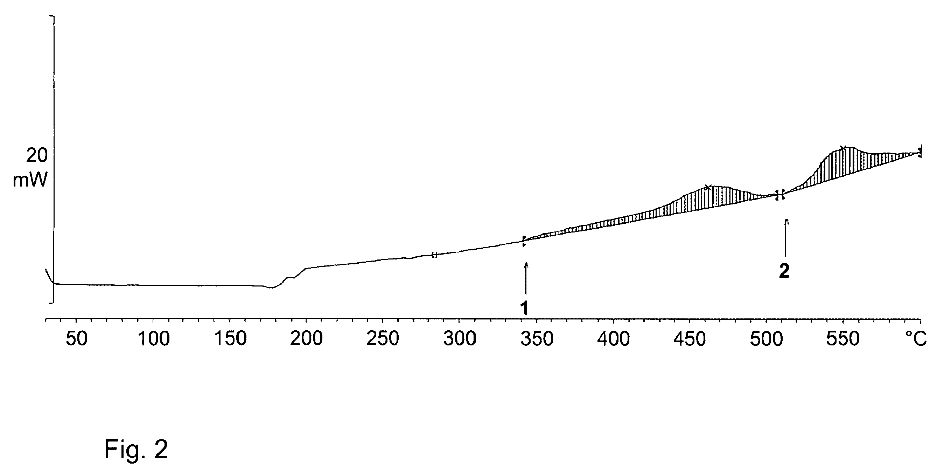

- Figur 2: zeigt die Wärmeentwicklung (in der Einheit mW, aufgetragen auf der Hochwertachse = y-Achse), aufgetragen über der Temperatur (in der Einheit °C, aufgetragen auf der Rechtswertachse = x-Achse), die im DSC-Versuch gemäß Beispiel 5 erhalten wurde. Der von der Hochwertachse überspannte Bereich beträgt 20 mW. Die Einteilung der Rechtswertachse erfolgt in Schritten von 10 °C, wobei die Rechtswertachse bei 30 °C beginnt und bei 600 °C endet. Was die Durchführung des Versuchs anbelangt, sei auf die entsprechenden Ausführungen in Beispiel 5 verwiesen. Die Bezugszeichen haben folgende Bedeutung:
1 Niedrigste Onset-Temperatur für den Reaktoraustrag (345 °C) für die einsetzende Zersetzungsreaktion
2 Onset-Temperatur (515 °C) für die zweite Reaktion
- Figur 3: zeigt die gemäß Beispiel 6 geschätzte adiabatische Induktionszeit in Abhängigkeit von der Temperatur. Auf der Rechtswertachste (=x-Achse) ist die Temperatur in °C aufgetragen, auf der Hochwertachse (= y-Achse) ist die αdiabatische Induktionszeit in Stunden aufgetragen. Zur Bestimmung der geschätzten Induktionszeit sie auf die Ausführungen in Beispiel 6 verwiesen.

### Beispiele:

### Beispiel 1

Es wird die Versuchsanlage gemäß Figur 1 eingesetzt.

Durch Strom 2 wird das frische Cyclopenten-Feed mit 116,4 g/h zudosiert. Dieser stammt aus der Destillation eines C₅-Schnitts eines Steamcrackers und hat folgende Zusammensetzung (Gew.-%): Cyclopenten (ca. 95,1 %), Cyclopentan (ca. 3,4 %), 2-Methyl-2-buten (ca. 1,2 %).

Dieser Strom 2 wird zunächst mit Strom 3 gemischt (Rück-Cyclopenten), um einen Strom zu erzeugen, der folgende Zusammensetzung hat: Cyclopenten (ca. 46,3 %), Cyclopentan (ca. 51,9 %), 2-Methyl-2-buten (ca. 0,9 %), 2,2-Dimethylbutan (ca. 0,81 %).

Dieser Strom wird dann mit einer Dosierpumpe zum Reaktor dosiert (Mengenstrom: ca. 2076 g/h). Durch Strom 1 wird flüssiges Distickstoffmonoxid (Gehalt an Distickstoffmonoxid > 99,8 Vol.-%, Firma Messer Griesheim) mit ca. 74 g/h zum Reaktor dosiert. Das molare Verhältnis Distickstoffmonoxid : Cyclopenten im Reaktorfeed beträgt 0,11 mol/mol. Der Reaktor besteht aus einem Rohr (Außendurchmesser = 60,3 mm, Wanddicke = 2,4 mm, Länge = 3500 mm) der mit dünnwandigen Raschig-Ringen aus Edelstahl (6×6×0,3 mm) gefüllt ist. Zur Bestimmung des Längstemperaturprofils wurde entlang der Reaktor Achse eine Thermohülse mit 16 mm Außendurchmesser installiert der mit einem 15-stufigen Thermoelement versehen ist. Das Reaktionsvolumen beträgt (abzüglich Füllkörpern und Thermohülse) inklusive Verbindungsstücke insgesamt ca. 7 L. Der Druck im Reaktor wird mit einer geeignete Druckhaltung am Reaktorausgang auf 100 bar eingestellt.

Das Rohr ist mit einem Isoliermantel aus einem nanoporösen anorganischen Schaum der Firma Microtherm versehen (zwei Lagen mit jeweils 50 mm Dicke). Um die Wärmeverluste noch weiter zu reduzieren, ist auf der Außenseite des Isoliermantels noch eine dreiteilige Begleitheizung installiert, die auf (von unten) 256, 275 und 317 °C eingestellt ist (durch Vorversuche ohne Zusatz von Distickstoffmonoxid wurde sichergestellt, dass die Wärmeverluste nur sehr gering sind. Wenn die Reaktoreingangstemperatur ca. 260°C beträgt, führen die verbleibenden Wärmeverluste zu einer Abkühlung um lediglich 3°C bis zum Reaktorausgang). In einem Vorversuch mit einem radioaktiven Marker, wurde die Verweilzeitverteilung des Reaktors bestimmt. Die ermittelte Bodensteinzahl hat den Wert von 117, was einer equivalenten Rührkesselkaskade mit 58 Rührkesseln entspricht.

Der Feedstrom wird über eine beheizte Eingangsdüse von unten in dem Reaktor gespeist in dem der Feedstrom auch aufgeheizt wird. Bei einer Reaktoreingangstemperatur von 239,5°C (gemessen an dem untersten Thermoelement in der Thermohülse) beträgt der Cyclopenten-Umsatz im geraden Durchgang 11% und der Distickstoffmonoxid-Umsatz ca. 96%. Die Selektivität zu Cyclopentanon bzgl. Cyclopenten beträgt 96,8%. Der Reaktoraustrag hat eine Temperatur von 295°C. Die adiabatische Temperaturerhöhung beträgt somit 55,5°C. Der Reaktoraustrag wird in einer zweistufige Entspannung auf 1 bar entspannt und abgekühlt. Die gasförmigen Komponenten werden abgetrennt und in einem Nachkühler (der bei + 5 °C betrieben wird) werden die darin enthaltenen Kohlenwasserstoffe möglichst vollständig auskondensiert.

Die flüssige Phase, die aus dem flüssigen Reaktoraustrag und des Kondensats wird in einer Destillationskolonne (Glockenbodenkolonne mit 20 Böden und flüssigem Seitenabzug) aufgetrennt. Als Sumpfprodukt erhält man 138,7 g/h eines Stroms mit folgender Zusammensetzung (Gew.-%): Cyclopentanon (ca. 95,3 %), Cyclopentan (ca. 0,8 %), 4-Pentenal (ca. 1,3 %), Cyclopentenoxid (ca. 0,37 %), Cyclopenten-Dimere (ca. 0,53 %), Cyclopenten (ca. 0,08 %). Das Seitenabzugsprodukt, das 45,6 % Cyclopenten enthält, wird als Strom 3 zum Reaktor zurückgeführt.

Die vereinigten Gasphasen (aus Entspannung & Kopf der Rückführkolonne, ca. 47,7 L/h) haben folgende Zusammensetzung: N₂ (81,1 Vol.-%), Distickstoffmonoxid (2,46 Vol.-%), Cyclopenten (0,96 Vol.-%), Cyclopentan (0,48 Vol.-%).

### Beispiel 2

Beispiel 1 wurde wiederholt, mit dem Unterschied, dass eine Reaktoreingangstemperatur von 255,4°C gewählt wurde. In diesem Fall betrug der Cyclopenten-Umsatz im geraden Durchgang ca. 12% und der Distickstoffmonoxid-Umsatz 99%. Der Reaktoraustrag hat eine Ausgangstemperatur von 313°C. Die adiabatische Temperaturerhöhung beträgt somit 57,6°C. Die Selektivität von Cyclopentanon bzgl. Cyclopenten betrug 94,8%.

### Beispiel 3

Beispiel 1 wurde wiederholt, mit dem Unterschied, dass eine Reaktoreingangstemperatur von 225,9°C gewählt wurde. In diesem Fall betrug der Cyclopenten-Umsatz im geraden Durchgang 9,7% und der Distickstoffmonoxid-Umsatz 83%. Der Reaktoraustrag hat eine Ausgangstemperatur von 277°C. Die adiabatische Temperaturerhöhung beträgt somit 51,1°C. Die Selektivität von Cyclopentanon bzgl. Cyclopenten betrug 95,2%.

### Beispiel 4

Um die Feinreinigung von Cyclopentanon zu demonstrieren wurden Austräge aus Beispiel 1, Beispiel 2 und andere analoge Versuche gesammelt und in eine Labortrennwandkolonne destilliert. Die Kolonne hatte einen Innendurchmesser von 43 mm und eine Höhe von 2,5 m und war mit einer Packung (Montz A3 1000) bestückt. Die Anzahl von theoretischen Trennstufen wurde mit einem Testgemisch in Vorversuche ermittelt und betrug 53 theoretische Trennstufen bei dem verwendeten F-Faktor. Die Trennwand war in dem Bereich zwischen 820 und 2100 mm oberhalb der Packungsunterkante gezogen und teilte die Kolonne genau in der Mitte durch. Feed und Seitenabzug waren beide 1300 mm oberhalb der Packungsunterkante platziert aber auf unterschiedlichen Seiten der Trennwand.

Der Kolonnenfeed (insgesamt 17,5 kg) hatte folgende Zusammensetzung (Gew.-%): Cyclopentanon (95,6), Cyclopentan (0,8), Cyclopenten (0,08), 4-Pentenal (1,3), Cyclopentenoxid 0,4), Isopropylmethylketon (0,2), Diethylketon (0,03), 2,2-Dimethylbutan (0,005) neben andere Komponenten wie Cyclopenten-Dimere, Cyclopentylcyclopentanon, Cyclopentenylcyclopentanone, Cyclobutancarbaldehyd, Cyclopropylacetaldehyd und andere nicht identifizierte Nebenkomponenten.

Die Destillation wurde bei Normaldruck durchgeführt mit einer Feedmenge von 0,33 kg/h und einem Rücklaufverhältnis von 90. Im Seitenabzug wurde Cyclopentanon mit einer Reinheit von 99,89% erhalten. Die Destillationsausbeute betrug 99,5% (bzgl. Cyclopentanon).

### Beispiel 5

Eine Probe (18,3 mg) aus dem bereits entspannten und entgasten Reaktoraustrag aus Beispiel 1 wurde unter Stickstoffatmosphäre in einem druckfesten V4A-Tiegel verschlossen und in eine geeignet Apparatur (Mettler TA 8000) wurde ein Differential-Scanning-Calorimetry (DSC) Versuch mit einer Temperaturrampe von 1 K/min durchgeführt. Das Ergebnis ist in Fig. 2 dargestellt.

Dabei ist in der Abbildung die Wärmeentwicklung in mW (y-Achse) über der Temperatur in °C (x-Achse) aufgetragen.

Die daraus ermittelte niedrigste Onset-Temperatur für den Reaktoraustrag beträgt demnach 345°C für die erste einsetzende Zersetzungsreaktion (in der Abbildung Fig. 2: 1). Dabei ergibt sich aus der Messung für die Wärme, die bei der ersten Zersetzungsreaktion frei wird, ein Wert von 263,7 J/g. Für die zweite Reaktion ergibt sich eine Onset-Temperatur von 515°C (in der Abbildung Fig. 2: 2). Für die zweite Reaktion ergibt sich aus der Messung für die Wärme, die bei der Zersetzungsreaktion frei wird, ein Wert von 208,7 J/g.

### Beispiel 6

Beispiel 5 wurde wiederholt mit unterschiedlichen Temperaturrampen von 0,3 K/min und 2,5 K/min. Die gewonnen Daten, zusammen mit den Daten aus Beispiel 5 wurden für die Ableitung einer formalen Zersetzungkinetik verwendet. Für die Auswertung wurde lediglich die Daten im Bereich zwischen 360 und 520°C verwendet, die mit dem Programm NETZSCH THERMOKINETICS analysiert wurden. Die beste Anpassung an den gemessenen Daten wurde mit einem Prout-Tompkins-Geschwindigkeitsansatz, der durch multivariante nichtlineare Regression an den Daten der DSC-Versuche angepasst wurde. Auf Basis des formalkinetischen Modells wurden für den Bereich 336 bis 380°C unter Annahme einer konstante Wärmekapazität von 2 J·g⁻¹·K⁻¹ die adiabatische Induktionszeit der Zersetzungsreaktion berechnet (hierzu wurde das Programm NETZSCH THERMSIM verwendet). Die damit geschätzte adiabatische Induktionszeit in Abhängigkeit der Temperatur ist in der Abbildung in Fig. 3 dargestellt. Dabei ist auf der x-Achse die Temperatur in °C aufgetragen und auf der y-Achse die adiabatische Induktionszeit in Stunden.

Demnach beträgt die adiabatische Induktionszeit 24 Stunden bei einer Anfangstemperatur des untersuchten Gemisches von ca. 350°C.

## Patentansprüche

1. Kontinuierliches Verfahren zur Herstellung von wenigstens einem monozyklischen Keton mit 4 bis 20 Kohlenstoffatomen durch Umsetzung bei einer Temperatur von 170 bis 340 °C eines Gemischs G1 enthaltend wenigstens ein monozyklisches Olefin mit 4 bis 20 Kohlenstoffatomen mit einem Gemisch G2 enthaltend wenigstens Distickstoffmonoxid, **dadurch gekennzeichnet, dass** diese Umsetzung adiabatisch durchgeführt wird und das Gemisch G1 und/oder G2 vor der Umsetzung zu wenigstens einem monozyklischen Keton mit 4 bis 20 Kohlenstoffatomen auf eine Temperatur von 170 bis 270 °C vorgewärmt werden, wobei die zum Vorwärmen von Gemisch G1 und/oder G2, notwendige Wärmeenergie zumindest teilweise aus dem Produktstrom des Verfahrens entnommen wird.

2. Verfahren nach Anspruch 1, wobei die zum Vorwärmen von Gemisch G1 notwendige Wärmeenergie zumindest teilweise aus dem Produktstrom des Verfahrens entnommen wird.

3. Verfahren nach Anspruch 1 oder 2, **dadurch gekennzeichnet, dass** ein Gemisch G1 eingesetzt wird, enthaltend 20 bis 98 Gew.-%, bevorzugt 30 bis 80 Gew.-%, besonders bevorzugt 40 bis 60 Gew.-%, jeweils bezogen auf Gemisch G1, wenigstens ein monozyklisches Olefin mit 4 bis 20 Kohlenstoffatomen und 2 bis 80 Gew.-%, bevorzugt 20 bis 70 Gew.-%, besonders bevorzugt 40 bis 60 Gew.-%, jeweils bezogen auf Gemisch G1, wenigstens einen weiteren Kohlenwasserstoff.

4. Verfahren nach einem der Ansprüche 1 bis 3, **dadurch gekennzeichnet, dass** das Gemisch G1 wenigstens ein monozyklisches Olefin enthält, ausgewählt aus der Gruppe bestehend aus Cyclopenten, Cyclohexen, Cyclohepten, Cycloocten und Mischungen davon.

5. Verfahren nach einem der Ansprüche 1 bis 4, **dadurch gekennzeichnet, dass** bei der Umsetzung das molare Verhältnis zwischen Distickstoffmonoxid und dem wenigstens einen monozyklischen Olefin zwischen 0,02 und 0,3 liegt.

6. Verfahren nach einem der Ansprüche 1 bis 5, **dadurch gekennzeichnet, dass** die adiabatische Temperaturerhöhung im Reaktor zwischen 10 und 140 °C beträgt.

7. Verfahren nach einem der Ansprüche 1 bis 6, **dadurch gekennzeichnet, dass** die adiabatische Temperaturerhöhung im Reaktor zwischen 25 und 100 °C beträgt.

8. Verfahren nach einem der Ansprüche 1 bis 7, **dadurch gekennzeichnet, dass** die Reaktorausgangstemperatur unterhalb der Onset-Temperatur für die Zersetzung des Produktgemisches liegt.

9. Verfahren nach einem der Ansprüche 1 bis 8, **dadurch gekennzeichnet, dass** die Reaktorausgangstemperatur mindestens 10 K unterhalb der Temperatur liegt, bei der die adiabatische Induktionszeit des Produktgemisches 24 Stunden beträgt.

10. Verfahren nach einem der Ansprüche 1 bis 9, **dadurch gekennzeichnet, dass** es bei einem Reaktionsdruck von 60 bis 500 bar durchgeführt wird.

11. Verfahren nach einem der Ansprüche 1 bis 10, **dadurch gekennzeichnet, dass** das Gemisch G2 mindestens 75 Vol.-% Distickstoffmonoxid enthält.

12. Verfahren nach einem der Ansprüche 1 bis 11, **dadurch gekennzeichnet, dass** der Umsatz bezüglich Distickstoffmonoxid bei 80 bis 100% liegt.

13. Verfahren nach einem der Ansprüche 1 bis 12, wenigstens umfassend die folgenden Schritte:
(A) Umsetzung eines Gemischs G1 enthaltend wenigstens ein monozyklisches Olefin mit 4 bis 20 Kohlenstoffatomen mit einem Gemisch G2 enthaltend wenigstens Distickstoffmonoxid unter adiabatischen Bedingungen, um ein Reaktionsgemisch G3 zu erhalten und
(B) Abtrennen des wenigstens einen monozyklischen Ketons mit 4 bis 20 Kohlenstoffatomen aus dem in Schritt (A) erhaltenen Reaktionsgemisch G3.

14. Verfahren nach Anspruch 13, **dadurch gekennzeichnet, dass** in der Trennstufe (B) nicht umgesetztes monozyklisches Olefin mit 4 bis 20 Kohlenstoffatomen abgetrennt wird.

15. Verfahren nach Anspruch 14, **dadurch gekennzeichnet, dass** das in der Trennstufe (B) abgetrennte nicht umgesetzte monozyklische Olefin mit 4 bis 20 Kohlenstoffatomen in Stufe (A) des Verfahrens zurückgeführt wird.

16. Verfahren nach Anspruch 1, wobei wenigstens ein Teil des Produktstroms mit wenigstens einem Teil von Gemisch G1 in einem Wärmetauscher in Kontakt gebracht wird.

## Claims

1. A continuous process for preparing at least one monocyclic ketone having from 4 to 20 carbon atoms by reacting, at a temperature of from 170 to 340°C, a mixture G1 comprising at least one monocyclic olefin having from 4 to 20 carbon atoms with a mixture G2 comprising at least dinitrogen monoxide, which comprises performing said reaction adiabatically and preheating the mixture G1 and/or G2 to a temperature of from 170 to 270°C before the reaction to give at least one monocyclic ketone having from 4 to 20 carbon atoms, wherein the thermal energy needed to preheat mixture G1 and/or G2 is withdrawn at least partly from the product stream of the process.

2. The process according to claim 1, wherein the thermal energy needed to preheat mixture G1 is withdrawn at least partly from the product stream of the process.

3. The process according to claim 1 or 2, wherein a mixture G1 is used comprising from 20 to 98% by weight, preferably from 30 to 80% by weight, more preferably from 40 to 60% by weight, based in each case on mixture G1, of at least one monocyclic olefin having from 4 to 20 carbon atoms, and from 2 to 80% by weight, preferably from 20 to 70% by weight, more preferably from 40 to 60% by weight, based in each case on mixture G1, of at least one further hydrocarbon.

4. The process according to any one of claims 1 to 3, wherein the mixture G1 comprises at least one monocyclic olefin selected from the group consisting of cyclopentene, cyclohexene, cycloheptene, cyclooctene and mixtures thereof.

5. The process according to any one of claims 1 to 4, wherein the molar ratio between dinitrogen monoxide and the at least one monocyclic olefin in the reaction is between 0.02 and 0.3.

6. The process according to any one of claims 1 to 5, wherein the adiabatic temperature increase in the reactor is between 10 and 140°C.

7. The process according to any one of claims 1 to 6, wherein the adiabatic temperature increase in the reactor is between 25 and 100°C.

8. The process according to any one of claims 1 to 7, wherein the reactor outlet temperature is below the onset temperature for the decomposition of the product mixture.

9. The process according to any one of claims 1 to 8, wherein the reactor outlet temperature is at least 10 K below the temperature at which the adiabatic induction time of the product mixture is 24 hours.

10. The process according to any one of claims 1 to 9, which is performed at a reaction pressure of from 60 to 500 bar.

11. The process according to any one of claims 1 to 10, wherein the mixture G2 comprises at least 75% by volume of dinitrogen monoxide.

12. The process according to any one of claims 1 to 11, wherein the conversion based on dinitrogen monoxide is from 80 to 100%.

13. The process according to any one of claims 1 to 12, at least comprising the following steps:
(A) reacting a mixture G1 comprising at least one monocyclic olefin having from 4 to 20 carbon atoms with a mixture G2 comprising at least dinitrogen monoxide under adiabatic conditions in order to obtain a reaction mixture G3 and
(B) separating the at least one monocyclic ketone having from 4 to 20 carbon atoms from the reaction mixture G3 obtained in step (A).

14. The process according to claim 13, wherein unconverted monocyclic olefin having from 4 to 20 carbon atoms is removed in the separation stage (B).

15. The process according to claim 14, wherein the unconverted monocyclic olefin having from 4 to 20 carbon atoms separated in the separation stage (B) is recycled into stage (A) of the process.

16. The process according to claim 1, wherein at least a portion of the product stream is contacted with at least a portion of mixture G1 in a heat exchanger.

## Revendications

1. Procédé continu pour la préparation d'au moins une cétone monocyclique comprenant 4 à 20 atomes de carbone par transformation, à une température de 170 à 340°C, d'un mélange G1 contenant au moins une oléfine monocyclique comprenant 4 à 20 atomes de carbone avec un mélange G2 contenant au moins du protoxyde d'azote, **caractérisé en ce que** cette transformation est réalisée de manière adiabatique et les mélanges G1 et/ou G2 sont préchauffés avant la transformation en au moins une cétone monocyclique comprenant 4 à 20 atomes de carbone à une température de 170 à 270°C, l'énergie thermique nécessaire pour le préchauffage des mélanges G1 et/ou G2 étant prélevée au moins partiellement du flux de produits du procédé.

2. Procédé selon la revendication 1, l'énergie thermique nécessaire pour le préchauffage du mélange G1 étant prélevée au moins partiellement du flux de produits du procédé.

3. Procédé selon la revendication 1 ou 2, **caractérisé en ce qu'**on utilise un mélange G1 contenant 20 à 98% en poids, de préférence 30 à 80% en poids, de manière particulièrement préférée 40 à 60% en poids, à chaque fois par rapport au mélange G1, d'au moins une oléfine monocyclique comprenant 4 à 20 atomes de carbone et 2 à 80% en poids, de préférence 20 à 70% en poids, de manière particulièrement préférée 40 à 60% en poids, à chaque fois par rapport au mélange G1, d'au moins un autre hydrocarbure.

4. Procédé selon l'une quelconque des revendications 1 à 3, **caractérisé en ce que** le mélange G1 contient au moins une oléfine monocyclique choisie dans le groupe constitué par le cyclopentène, le cyclohexène, le cycloheptène, le cyclooctène et leurs mélanges.

5. Procédé selon l'une quelconque des revendications 1 à 4, **caractérisé en ce que**, lors de la transformation, le rapport molaire entre le protoxyde d'azote et ladite au moins une oléfine monocyclique est situé entre 0,02 et 0,3.

6. Procédé selon l'une quelconque des revendications 1 à 5, **caractérisé en ce que** l'augmentation adiabatique de la température dans le réacteur est située entre 10 et 140°C.

7. Procédé selon l'une quelconque des revendications 1 à 6, **caractérisé en ce que** l'augmentation adiabatique de la température dans le réacteur est située entre 25 et 100°C.

8. Procédé selon l'une quelconque des revendications 1 à 7, **caractérisé en ce que** la température de sortie du réacteur est inférieure à la température de démarrage de la décomposition du mélange de produits.

9. Procédé selon l'une quelconque des revendications 1 à 8, **caractérisé en ce que** la température de sortie du réacteur est inférieure d'au moins 10 K à la température à laquelle le temps d'induction adiabatique du mélange de produits est de 24 heures.

10. Procédé selon l'une quelconque des revendications 1 à 9, **caractérisé en ce qu'**il est réalisé à une pression de réaction de 60 à 500 bars.

11. Procédé selon l'une quelconque des revendications 1 à 10, **caractérisé en ce que** le mélange G2 contient au moins 75% en volume de protoxyde d'azote.

12. Procédé selon l'une quelconque des revendications 1 à 11, **caractérisé en ce que** la conversion par rapport au protoxyde d'azote est de 80 à 100%.

13. Procédé selon l'une quelconque des revendications 1 à 12, comprenant au moins les étapes suivantes :
(A) transformation d'un mélange G1 contenant au moins une oléfine monocyclique comprenant 4 à 20 atomes de carbone avec un mélange G2 contenant au moins du protoxyde d'azote dans des conditions adiabatiques pour obtenir un mélange réactionnel G3 et
(B) séparation de ladite au moins une cétone monocyclique comprenant 4 à 20 atomes de carbone du mélange réactionnel G3 obtenu dans l'étape (A).

14. Procédé selon la revendication 13, **caractérisé en ce que**, dans l'étape de séparation (B), l'oléfine monocyclique, comprenant 4 à 20 atomes de carbone, non transformée, est séparée.

15. Procédé selon la revendication 14, **caractérisé en ce que** l'oléfine monocyclique, comprenant 4 à 20 atomes de carbone, non transformée, séparée dans l'étape de séparation (B) est recyclée dans l'étape (A) du procédé.

16. Procédé selon la revendication 1, au moins une partie du flux de produits étant mise en contact avec au moins une partie du mélange G1 dans un échangeur thermique.
